# EUROPÄISCHE PATENTSCHRIFT

(11) **EP 3 694 420 B1**
(45) Veröffentlichungstag und Bekanntmachung des Hinweises auf die Patenterteilung: **13.04.2022**
(21) Anmeldenummer: 18799431.4
(22) Anmeldetag: 10.10.2018
(51) Int. Cl.: A61B 17/34, A61B 17/22, A61B 17/30, A61M 60/148, A61B 17/00

(54) **PERIKARDGREIFER**
PERICARDIUM GRIPPER
OUTIL DE PRÉHENSION DE PÉRICARDE

(30) Priorität: 12.10.2017 DE 102017009488
(43) Veröffentlichungstag der Anmeldung: 19.08.2020
(73) Patentinhaber: Christoph Miethke GmbH & Co. KG, 14469 Potsdam (DE)
(72) Erfinder: MIETHKE, Christoph, 14467 Potsdam (DE); THIEME, Lucas, 14467 Berlin (DE); KLETTNIG, Tim, 14473 Potsdam (DE)
(74) Vertreter: Greiche, Albert
(86) Internationale Anmeldenummer: PCT/EP2018/000468
(87) Internationale Veröffentlichungsnummer: WO 2019/072412

(56) Entgegenhaltungen:
- WO-A1-2013/190967
- WO-A1-2013/190968
- WO-A1-2016/025850
- US-A1- 2017 143 322

## Beschreibung

Die Erfindung betrifft eine Vorrichtung zum Greifen von Perikard nach Anspruch 1 umfassend mindestens eine innere Röhre und mindestens eine äußere Röhre, wobei die äußere Röhre mindestens einen äußeren Durchmesser aufweist und wobei die innere Röhre mindestens einen inneren Durchmesser aufweist, wobei der äußere Durchmesser größer ausgebildet ist als der innere Durchmesser und die innere Röhre an mindestens einem Innenröhrenende mindestens eine Innenröhrenendfläche aufweist, und wobei die Innenröhrenendfläche mindestens eine erste Oberflächenstruktur aufweist und wobei die äußere Röhre an mindestens einem Außenröhrende mindestens eine Außenröhrenendfläche aufweist, und wobei die Außenröhrendfläche mindestens eine zweite Oberflächenstruktur aufweist, und die erste Oberflächenstruktur und die zweite Oberflächenstruktur unterschiedlich ausgebildet sind.

Das Herz ist ein notwendiges Organ zur Lebensfähigkeit verschiedener Spezies. Auch der Spezies Mensch dient sein periodisches Kontrahieren und Relaxieren, sein Schlagen, einem Fördern von Blut durch dessen Körper. Seinen Dienst modelliert die Wissenschaft als Verdrängerpumpe, sein Rhythmus parametrisiert sie darin als Öffnen und Schließen von Ventilklappen, um sein Wirken zu verstehen.

Die Lehre vom Wirken des Herzens in Struktur und Funktion, sowie ihr Wirken im Zusammenspiel mit anderen Organen ist die Kardiologie. Sie subsummiert auch Dysfunktionen am Herzen. In Folge von Alterung, Behandlung oder Erkrankung kann die Schlagkraft des Herzens verringert sein. Ursachen dieser sogenannten Herzinsuffizienz können Bluthochdruck, Herzklappenfehler oder Verkalkungen von Herzkranzgefäßen sein.

Sarah Zubke führt in ihrer Masterarbeit: "Entwicklung eines Herzbeutel Zugangs für die Einbringung eines das Herz unterstützenden Implantats", Herbst 2017, Fachgebiet Medizintechnik, Technische Universität Berlin (Quelle 1) Grundlagen zur Herzinsuffizienz aus.

Ihrer Arbeit nach werden terminologisch Insuffizienzen gemäß der European Society of Cardiology nach drei Formen differenziert, die sogenannten: systolische und diastolische Insuffizienz sowie Herzinsuffizienz mit mittlerer linksventrikulärer Ejektionsfraktion. Gemäß der Arbeit von Frau Zubke wurden im Jahr 2014 nach dem Statistischem Bundesamt etwa 430.000 Patienten mit einer Herzinsuffizienz und ca. 36.000 daran Verstorbene erfasst.

Einem Therapieren von Herzinsuffizienz soll heute eine Menge an Maßnahmen dienen. Bekannte Elemente dieser Menge sind, sequenziert entlang eines Maßstabs: Herztransplantation, Herzschrittmacher oder Medikamente. Die Sequenz nimmt mit dem Schweregrad der Herzinsuffizienz ab.

Auch ein Therapieren mittels einer Herzunterstützung ist ein Element, das auf dem Maßstab neben dem Element Herztransplantation eingeordnet werden kann. Beide Elemente differieren im Kriterium ihrer Einsatzdauer. Herzunterstützende Therapien avisieren Einsatzdauern von Stunden oder Tagen, Herztransplantationsgestützte Therapien hingegen Monate oder Jahre.

Hinsichtlich einer Herzunterstützung umfasst der Stand der Technik: intraortale Ballonblutpumpen, perkutan intravasale Pumpsysteme - sogenannte Blutpumpen oder perkutane Ummantelungsvorrichtungen.

Die US 6544216B1 lehrt eine intrakardiale Blutpumpe. Sie ist schlauchartig ausgestaltet, ihr erstes Ende in den rechten Vorhof und ihr zweites Ende in die Lungenarterie - durch die Lungenherzklappe operiert. Ein Abschnitt ihrer schlauchartigen Gestalt umfasst eine Pumpsektion. Daher pumpt die intrakardiale Blutpumpe im Betrieb Blut aus dem rechten Vorhof in die Lungenarterie.

Blut durchströmt die Pumpsektion, es tritt radial am ersten Ende in sie ein, verlässt sie am zweiten Ende hingegen axial.

Weil das Blut die Pumpsektion durchströmt, treten Oberflächeneffekte zwischen einzelnen Blutzellenoberflächen und innenliegenden Pumpsektionsflächen auf. Diese Oberflächeneffekte, zum Beispiel Reibung schädigen die Blutzellen.

Ein Nachteil der intrakardialen Blutpumpe ist somit ihre blutzellschädigende Wirkung.

In der WO2017134304A1 ist eine Technik einer Pumpe, die durch passive Magnetwirkung unterstützt wird, beschrieben. Diese -Blut- Pumpe fördert Fluid von ihrem Einlass zu ihrem Auslass, in dem sie einen Fördererdruck aufbaut, dessen Druckkraft eine die Blutpumpe verschließende Verschlusskraft überwindet.

Die Verschlusskraft resultiert dabei aus einem passiven magnetischen Lagern des Rotors in der Blutpumpe, wodurch dieser axial in eine Richtung zumindest passiv magnetisch angezogen oder abgestoßen wird. Weil er magnetisch angezogen oder abgestoßen wird, wird der Rotor in einen Sitz zwangsgeführt.

Die Druckkraft resultiert aus einem Übertragen von Rotationsenergie mittels Schaufeln vom Rotor auf das ihn umschließenden Blut. Durch sein Rotieren wird Blut in eine Richtung geschaufelt - ein Förderstrom aus Blut- vorgeschoben.

Wenn die Druckkraft der Verschlusskraft der Richtung nach entgegensteht und der Stärke nach größer ist, fördert die Blutpumpe.

Die Schaufeln berühren das Blut nicht nur, sondern Erzeugen infolge ihrer Rotationsgeschwindigkeit Reibung zwischen Blutzellenoberflächen und den Schaufelflächen. Somit gefährden sie die Blutzellen.

Eine Marktteilnehmerin beschreibt auf ihrer Webpage: http://www.cardiobridge.com/technology/ am 14.09.2017 eine Technologie namens "10F-Reitan Cathter". Gemäß ihrer eigenen Auskunft handelt es sich dabei um eine subkutan und intraarteriell arbeitendes Kurzzeitunterstützungsgerät ("short term intraaortic percutaneous circulatory support"). Im Betrieb ist das Produkt in die Lungenarterie eingebracht und vom Herzen beanstandet. Das Produkt umfasst ein auf gespanntes Drahtnetz in welchem ein Propeller rotiert. Das Drahtnetz balloniert dabei den ihn umgebenden Abschnitt der Lungenarterie. Der Propeller liegt im Drahtnetz quer zur Richtung des Blutflusses. Seine Rotation beschleunigt somit den Blutfluss, in dem kinetische Energie auf diesen übertragen wird. Je höher die Übertragung an kinetische Energie, desto höher ist die Substitution der Förderleistung des Herzens.

Allerdings ist der Propeller dabei in direktem Blutkontakt mit der Folge einer Blutzellenzerstörung.

Allen hier vorgestellten Blutpumpen ist der Nachteil gemeinsam, Blutzellen zu schädigen, in der Regel zu zerstören. Dieser Nachteil ist im Stand der Technik als sogenannte Hämolyserate bekannt. Sie beschreibt das Verhältnis aus zerstörten zu nicht zerstörten Blutzellen bzw. gepumptem Blutvolumen.

Die EP102008018919A1 schlägt eine Ummantelungsvorrichtung zur Unterstützung und/oder Übernahme der Pumpfunktion des Herzens vor. Dadurch soll sie in Bedarfsfällen, zum Beispiel während einer akuten Herzinsuffizienz eine gerichtete zyklische Kompression und Dekompression auf das Herz aufbringen, um die Herzpumpleistung zu unterstützen. Dem Aufbringen liegt der Gedanke zugrunde, dass Herz mit einem Mantel zu umschließen, dessen Mantelvolumen kompressibel ist. Ihm folgend werden seine Volumenänderungen als gerichtete Kraft auf die Herzoberfläche aufgebracht. Ihn fassend, setzt er ein Einbringen des Mantels in den Patienten voraus. Diese Voraussetzung führt der Vorschlag weiter aus, in dem die Manteleigenschaft der Kompressibilität ausgenutzt wird, die Umwandlungsvorrichtung faltbar auszuführen. Diese Ausführung ist wenig invasiv und wenig anspruchsvoll.

Ihre zwingende Funktionsvoraussetzung das Herz zu ummanteln, ist ihr wesentlicher Nachteil. Ein Ummanteln im Bedarfsfall bedeutet, ein Ummanteln unter Zeitdruck und unter ungünstigen Bedingungen. Beide Bedeutungsgehalte erschweren ein korrektes Anwenden der Umwandlungsvorrichtung in der Praxis.

Im Stand der Technik sind verschiedene Hilfsmittel bekannt, die einer behandelnden Person, zum Beispiel einem Arzt in der Behandlung einer Herzinsuffizienz zur Verfügung stehen. Eine Mehrzahl von Hilfsmitteln hilft dem Arzt in einer Operation medizinische Geräte, wie Blutpumpen an ihren Bestimmungsort im menschlichen Körper zu verbringen.

Bekannte Hilfsmittel sind Skalpelle, Kanülen oder Zangen.

Skalpelle sind sehr scharfe Klingen. Sie werden vom Arzt von Hand geführt und erzwingen von ihm handgeführte, freigeformte Schnitt. Soll mit einem Skalpell bis zu einem tiefliegenden Bestimmungsort im menschlichen Körper vorgedrungen werden, muss eine offenliegende Klinge vom Arzt durch den Körper geführt werden.

Ihre scharfe Klinge wird dem Skalpell zum Nachteil. Sie setzt dem menschlichen Körper das Risiko gefährlicher Schnitte aus.

Kanülen sind medizinische Nadeln, umfassen zum Beispiel Hohl-, und Injektionsnadeln. Sie werden vorzugsweise verwendet, um Flüssigkeiten in den menschlichen Körper zu spritzen oder aus ihm abzuziehen. Sie werden dafür in den Körper eingestochen, in dem sie durch die Haut, innere Häute, Gewebe, Gewebeabschnitt, Organe vorgeschoben werden. Dieser Vorschub wird ermöglicht und erleichtert, weil die Nadelspitze scharf ausgeführt, in der Regel angeschliffen ist.

Ihre scharfe Nadelspitze wird auch der Kanüle zum Nachteil. Wird die Kanüle zu tief - beispielsweise über den Bestimmungsort hinaus- in den Körper vorgeschoben, werden unbeabsichtigt, teils unbemerkt wichtige Körperteile verletzt.

Medizinische Zangen helfen dem Arzt Körperteile festzuhalten. Wird mit Ihnen falsch oder zu stark gegriffen, weisen Sie den Nachteil auf Gewebe einzureisen.

Die Anatomie differenziert das Herz von dessen ihn umgebenden Beutel. Diesen Beutelsack- bezeichnete sie als Herzbeutel, in der Fachsprache des Arztes als Perikard.

Einen Überblick zu Erkrankungen des Perikards gibt die ESC- Richtlinie (ESC Guideline) "Guideline an the Diagnosis and Management of Pericardial Diseases", European Heart Journal 2004.

Ein minimales Öffnen des Perikards, dessen punktuelles Durchstechen, bzw. Durchpieksen wird als Perikardpunktion bezeichnet. Sie differenziert das so genannte nasse vom trockenen Punktieren. Dazu erhebt sie die An-, oder Abwesenheit eines zwischen dem Herz und dem Herzbeutel liegenden Fluid-, oder Flüssigkeitspolster, in lingua medicus Erguss zum Kriterium.

Liegt ein Erguss vor, sodass ein Arzt von außen durch das Perikard in den Erguss punktiert, punktiert er nass. Liegt kein Erguss vor, liegt das Perikard also nah oder auf dem Herz, punktiert er trocken.

Heute wird das Herz mechanisch behandelt, wenn das Perikard nass punktiert werden kann. Dieser Fall liegt vor, wenn der Erguss ein ungefähres Minimalvolumen von ca. 25ml ausfüllt, denn dieses Minimalvolumen kompensiert ein mögliches Fehlführen eines Behandlungswerkzeugs -einer Kanüle oder Nadel- durch den behandelnden Arzt. Der Erguss schützt folglich vor einem gewissen Grad des Verrutschens, Abrutschens oder Verschätzens im Moment der Punktion.

Eine Vorrichtung zum Punktieren wird z. B. in WO 2013/190968 A1 beschrieben. Aus ihr ist ein Zugangsport bekannt, der an einer biologischen Membran befestigt ist, wie z.B. dem Perikard, in einem Zustand, in dem es die biologische Membran durchdringt. Vorgesehen ist eine Zugangsöffnung mit einer inneren Röhre und einer äußeren Röhre, die so zusammengefügt sind, dass sie relativ zueinander in einer axialen Richtung beweglich sind, wobei mindestens eine von der inneren Röhre und der äußeren Röhre Halteflächen mit im Wesentlichen komplementären Formen aufweist, die durch relative Bewegung der inneren Röhre und der äußeren Röhre dosismäßig aufeinander zu oder voneinander weggebracht werden.

Wird punktiert, wird daher üblicherweise ein röntgenbildgebendes Verfahren verwand, um Erstens einen Vorschub des Behandlungswerkzeugs optisch verfolgen zu können, und um Zweitens den Moment der Punktion detektieren zu können.

Dabei wird, z.B. bei Verwendung einer Nadel durch Spitze ein Röntgenkontrastmittel appliziert. Dieses bildet innerhalb eines Gewebes einen Flüssigkeitspunkt vor der Nadelspitze, im Moment der Punktion, in der Kavität des Perikards hingegen einen Schleier. Zerfließt ein Flüssigkeitspunkt zu einem Schleier, lässt sich somit im Röntgenbild unterscheiden, ob das Perikard punktiert ist oder nicht.

In der EP1956963B1 ist eine Anordnung zur Führung von Instrumenten erläutert. Sie verweist als Beispiele von Instrumenten auf Endoskope, Entnahme- oder Montagewerkzeuge oder Werkzeuge zur optischen Abbildung ausgesuchter Bereiche in Hohlräumen. Die Anordnung soll im allgemeinen Fall ein Führen von Instrumenten relativ zu mindestens einem Teil der Wände eines Hohlraums, im speziellen Fall ein Führen medizinischer oder tiermedizinischer Instrumente in Hohlräumen in Lebewesen ermöglichen. Sie löst den speziellen Fall, in dem sie die Anordnung segmentiert, sodass sie eine Gliedkette ist, deren Gelenke abknickbar miteinander verbunden sowie in der Länge einzelner Segmente oder mehrerer verbundener Segmente veränderbar ausgeführt sind. Aufgrund dieser Lösung sieht sie deren Nutzen im erstmaligen Ermöglichen: definierter Untersuchungen, zuverlässiger Punktionen oder anderweitiger Manipulationen.

Der sogenannte Marburger-Attacher befähigt nach Auskunft eines Online-Innovationsreport, "Marburger-Attacher revolutioniert Herzbeutel Punktion", vom 09.03.2005 auf einer Unterseite der Webpage, "www.innovation-report.de" einen Anwender zum "gezielten und signalüberwachten minimal-invasiven Manipulieren von Organen".

Der Marburger-Attacher gestaltet das Phänomen aus in einem geschützten Raum Perikard zu punktieren, in dem in eines seiner Enden ein Volumen ausgenommen ist, in das Perikard gesaugt wird, so dass durch dessen Halt auf Grund dieser Saugkraft das Perikard vom Herzen weggedreht werden kann, um es sicher zu punktieren.

Das Schaffen eines geschützten Raums für eine Punktion ist aufwendig. Ein Bereitstellen eines solchen Raums resultiert in einem Bereitstellen dessen Volumens. Diese macht den Marburger-Attacher groß und unhandlich.

Zusammenfassend sind im Stand der Technik Wirkmechanismen bekannt, denen ein Ansaugen des Herzbeutels zugrunde liegt.

Nachteil des Stands der Technik ist, dass er einen größeren invasiven Zugriff auf das Herz voraussetzt.

In der US9585689B2 ist eine Lehre eines Zugangs - einer Schleuse veröffentlicht. Diese bietet damit eine definierte Passage im Allgemeinen durch eine biologische Membran, - und im Detail durch das Perikard an. Die Schleuse umfasst als Außenteil zwei ineinandergesteckte Röhren, und ein im Außenteil geführtes Innenteil. Sie wird in eine Punktion geschoben, weil diese in einem ersten Schritt mit einer Nadel punktiert, durch diese in einem zweiten Schritt ein Draht geführt, und in diese in einem dritten Schritt: Innenteil, innere-, und äußere Röhre, - die Schleuse über den Draht geführt wurden, weil der Draht durch die Schleuse getunnelt ist. Dem vorzeitigen Schieben der Schleuse in die Punktion, folgt nachzeitig ein Fixieren des Perikards in der Schleuse. Weil die innere Röhre axial gegen die Vorschubrichtung zurückgezogen werden kann, kann ein Abstand zwischen der inneren- und der äußeren Röhre verkleinert werden. Weil dieser Abstand eine Nutbreite beschreibt, resultiert aus einem Verkleinern ein Zusammenziehen der Nut. Weil vorgesehen ist, dass die Nut in der Punktion liegt, klemmt die Nut die Punktion umliegendes Perikard ein, wenn die Nut verkleinert wird.

Ein Gebrauchen der Schleuse setzt eine Perikardpunktion voraus. Unglücklicherweise weißt die Schleuse keine Funktionalität auf, um das Perikard zu punktieren, so dass die Punktion mittels eines zweiten Werkzeuges durchzuführen ist. Ein Einbringen eines zweiten Werkzeugs in den menschlichen Körper, ist ein Einbringen einer zweiten Verunreinigungsquelle. Daraus resultiert ein Nachteil des nächstkommenden Stands der Technik, er erhöht die Infektionsgefahr.

Ein Gebrauchen der Schleuse setzt ferner deren Fixation voraus. Weil diese, Resultat eines Einklemmens von Perikard in einer Nut ist, ist die Schleuse von einer behandelnden Person so lange zu manipulieren, bis die Nut in der Punktion liegt. Ein Manipulieren in der Enge und Dunkelheit des menschlichen Körpers durch einen minimalinvasiven Schnitt ist kompliziert und gefährdet durch diese Komplexität einen Therapieerfolg.

Nach dem Gebrauchen der Schleuse ist sie zwingend aus dem menschlichen Körper zu entfernen. Regelmäßig ist die Schleuse dazu zu ziehen. Weil Perikard im korrekten Gebrauch der Schleuse in der Nut ist, bewirkt ein Herausziehen der Schleuse ein Aufreißen der Punktion.

Aufgabe der Erfindung ist, ein sanftes Einschleusen medizinischer Geräte in menschliche Hohlräume zu schaffen. Sie wird von den Merkmalen des Hauptanspruchs gelöst. Die Unteransprüche beschreiben bevorzugte Ausführungsformen der Erfindung.

Diese Aufgabe wird von einer Vorrichtung zum Greifen von Perikard nach Anspruch 1 gelöst.

Erster Vorteil: In Ihrer Masterarbeit -Quelle 1- arbeitet Frau Sarah Zubke die Bedeutung der Geschwindigkeit für das Patientenwohl eines Einschleusens von medizinischen Geräten am Beispiel eines Einführbestecks heraus. Ihren Ergebnissen nach sind Vorrichtungen zum Einbringen von Führungsdrähten für die klinische Praxis ungeeignet, wenn diese nicht ein Einschleusen innerhalb von 55 Minuten, vorzugsweise 30 Minuten, insbesondere 10 Minuten ermöglichen. Ihre Ergebnisse erheben die Einschleusegeschwindigkeit somit zu einem Kriterium für eine Beurteilung von Lösungsvorschlägen zum Stand der Technik. Ein Umkehren der Ergebnisse von Frau Zubke lässt somit den Schluss zu, dass ein Verfahren und eine Vorrichtung an Wert gewinnen, wenn sie ein Einschleusen in kurzer Zeitspanne, - je kürzer, desto besser - von medizinischen Geräten in das Perikard ermöglichen.

Mittels der erfindungsgemäßen Vorrichtung lässt sich ein medizinisches Gerät schnell in das Perikard einbringen. Dieser Schnelligkeit tragen sowohl dessen unkomplizierte Verfahrensschritte als auch der Verfahrensschritt des Herzbeutelgreifens bei.

In dem mit mindestens einem Vorrichtungsende durch den menschlichen Körper vorgedrungen wird bis mit diesem der Herzbeutel, das Herz oder eine auf dem Herz angeordnete Schicht berührt wird, lassen sich alle drei Vorgenannten schnell finden. Das Vordringen wird besonders schnell, weil es für eine behandelnde Person noch einfacher wird, wenn die Vorrichtung starr ausgebildet ist, sodass das Vorrichtungsende durch den menschlichen Körper gesteckt werden kann.

Neben anderen kann als Einschleusezeit folgende Definition vorgenommen werden: Die Zeitspanne zwischen dem Zeitpunkt des Öffnens eines menschlichen Körpers und dem Zeitpunkt einer sollgemäßen Inbetriebnahme eines temporären Herzunterstützungssystems.

Zweiter Vorteil: Ein Greifen des Herzbeutels, des Herzens oder einer auf dem Herzbeutel liegenden Schicht mit mindestens einem Außenteilsende kann schnell erfolgen.

Dritter Vorteil: Indem die beanspruchte Vorrichtung dazu geeignet ist, Perikard zu greifen, weist sie den Vorteil auf, gegriffenes Perikard vom Herz beabstanden zu können. Der Abstand öffnet einen Raum zwischen Perikard und Herz, in den ein Punktion-, oder Schnittwerkzeug nach Durchdringen des Perikards eindringen kann, ohne dass das Durchdringen das Herz durch ein Eindringen des Werkzeugs unmittelbar gefährdet, denn das Werkzeug dringt mittelbar in den Raum. Vice versa ist der vierte Vorteil demnach das Erhöhen der Behandlungssicherheit.

Die Aufgabe wird von einer erfindungsgemäßen Vorrichtung zum Greifen von Perikard nach Anspruch 1 gelöst, diese Vorrichtung umfasst mindestens eine innere Röhre und mindestens eine äußere Röhre , wobei die äußere Röhre mindestens einen äußeren Durchmesser aufweist und wobei die innere Röhre mindestens einen inneren Durchmesser aufweist, wobei der äußere Durchmesser größer ausgebildet ist als der innere Durchmesser und die innere Röhre an mindestens einem Innenröhrenende mindestens eine Innenröhrenendfläche aufweist, und wobei die Innenröhrenendfläche mindestens eine erste Oberflächenstruktur aufweist und wobei die äußere Röhre an mindestens einem Außenröhrende mindestens eine Außenröhrenendfläche aufweist, und wobei die Außenröhrendfläche mindestens eine zweite Oberflächenstruktur aufweist, und die erste Oberflächenstruktur e und die zweite Oberflächenstruktur unterschiedlich ausgebildet sind, wobei die innere Röhre beweglich in der äußeren Röhre angeordnet ist.

Erster Vorrichtungsvorteil: Die erfindungsgemäße Vorrichtung folgt dem Grundsatz «zusammengesteckter Röhren, bzw. zusammengesteckter Schläuche», dessen eines Deduktiv deren kleines Volumen ist. Erst- die Existenz einer erfindungsgemäßen Vorrichtung kleinen Volumens kann eine behandelnde Person zu einem Verhalten minimalinvasiven Öffnens des menschlichen Körpers führen. Das Hinführen der behandelnden Person zu diesem patientenschonenden Verhalten ist folglich der erste Vorrichtungsvorteil. Neben einer Mehrzahl bekannter Sekundärvorteile, zum Beispiel schnelle Wundheilung einer Minimalinvasion, ist ihr Primärvorteil ihre schnelle Durchführung.

Zweiter Vorrichtungsvorteil: Überraschender Weise schont die erfindungsgemäße Vorrichtung einen Patienten weiter, weil sie einen Griff im ersten Versuch zulässt. Dieser Vorteil erschließt sich aus zwei Prämissen. Die Erste lautet, dass mit ihr menschliches Gewebe aller Art gegriffen werden kann. Die Zweite lautet, dass ein mehrfaches Greifen menschlichen Gewebes für einen Patienten belastend ist, dass also einmaliges Greifen menschlichen Gewebe schonend ist. Wenn also mit der erfindungsgemäßen Vorrichtung eine Gewebeart gegriffen wurde und ein einmaliges Greifen grundsätzlich schonender ist, muss die behandelnde Person nicht nach einer bestimmten Gewebeart greifen.

Dritter Vorrichtungsvorteil: Im Gebrauch der erfindungsgemäßen Vorrichtung ist ein Verdrängen von Fett von großem Zugewinn.

[A2] In einer bevorzugten Ausführungsform wird zum Greifen des Herzbeutels ein Außenteil gegen ein Innenteil verdreht. Das Verdrehen erfolgt durch ein Handhaben einer behandelnden Person, ist also in erster Folge mechanisch, ist also in zweiter Folge ein Greifen, und damit in der Gesamtfolge ein mechanisches Greifen. Dieses lässt phänomenologisch aus sich heraus ein Feedback an die behandelnde Person zu. Der Vorteil mechanischen Greifens ist dessen inhärentes Rückmelden des Greifens beim Greifen und des Griffs im Griff. Dieser Vorteil, das taktile Feedback begünstigt ein sicheres und schnelles Arbeiten durch die behandelnde Person.

[A3] In einer bevorzugten Ausführungsform wird zum Greifen des Herzbeutels mit dem Außenteil mindestens eine äußere Röhre gegen mindestens eine innere Röhre verdreht. Auf Grund eines Greifens dieser Art gewinnt das Greifen an Positions-, bzw. Lagekonstanz. Eine Voraussetzung dieser Greifart ist erfüllt, wenn ein Innenteil die erfindungsgemäße Vorrichtung gegenüber dem Perikard positioniert. Dann können Baugruppen des Außenteils - das über das Innenteil zwangsgeführt istmehrfach gegeneinander verdreht werden, denn die Konstanz der Lage der erfindungsgemäßen Vorrichtung wird nicht durch das Außenteil, sondern durch das Innenteil begründet.

[A4] In einer bevorzugten Ausführungsform wird zum Greifen des Herzbeutels eine erste Außenteilendfläche gegen eine zweite Außenteilendfläche verdreht, d.h. eine erste Stirnfläche gegen eine zweite Stirnfläche verdreht.

Wenn Flächen aneinander reiben, sind sie im Eingriff. Dieser Eingriff ist Folge aus einem - mikroskopischen- Verhaken ihrer Oberflächenstrukturen. Die Stärke des Eingriffs, die Haltekraft des Griffs ist proportional zum Verhaken. Aufgrund des Obersatzes: Je gradueller die Verdrehung, desto stärker das Verhaken und des Untersatzes: Je stärker das Verhaken, desto stärker die Haltekraft schließt im Modus ponens die Figur: Je gradueller die Verdrehung, desto stärker die Haltekraft. Der Vorteil eines Verdrehens von Außenteilendflächen ist ein Steigern von Haltekraft. Diese Steigerung wird noch begünstigt, wenn die Außenteilendflächen gegeneinander verdreht werden.

Werden Oberflächenstrukturen in die Außenteilende Flächen eingebracht, wird die Haltekraft weiter gesteigert. Vorteilhafterweise sind dabei Nuten, Riefen, Rillen, oder Züge in die Oberfläche eingebracht. Deren Struktur kann periodisch, gefächert oder überlappend sein.

Weil die Außenteilendflächen an einem Ende des Außenteils liegen, kann die Geometrie der Außenteilendflächen vorteilhafter Weise leicht geändert werden. Diese Veränderung kann ein Steigen der Haltekraft begünstigen, wenn ein Stellungswinkel zwischen dem Flächenvektor einer Außenteilendfläche und dem Außenteil vergrößert oder verkleinert wird.

[A5] In einer bevorzugten Ausführungsform wird der gegriffene Herzbeutel vom Herzen mittels der Vorrichtung mechanisch abgehoben.

Das Abheben beabstandet das Herz vom Herzbeutel. Dieser Sicherheitsabstand sichert das Herz vor einem absichtlichen oder unabsichtlichen Eindringen eines Punktion-, oder Schnittwerkzeuge durch eine behandelnde Person.

[A6] In einer bevorzugten Ausführungsform wird das Innenteil aus der Vorrichtung entfernt.

Das Entfernen gibt einen Durchlass im Außenteil frei, sodass ein Zugang zum Perikard geschaffen wird. Der Vorteil dieses Zugangs ist, dass durch ihn Werkzeuge zum Perikard geschleust werden können. Sind diese Werkzeug Punktions-, oder Schnittwerkzeuge kann am Perikard manipuliert werden, um dieses zu Öffnen.

Das Öffnen kann
- der Gestalt nach mindestens ein: Punkt-, Schlitz-, Stern-,
- der Gattung nach Nadeln, Schneiden, Stanzen,
- der Güte nach im Reim dabei nur Miethke:

Manuell, Intentional, Evident, Trennend, -Halt- Könnend im Ergebnis sein.

Der Vorteil des Öffnens ist schlicht, hat es als Phänomen: <<Trockenes Punktieren durch Mechanisches Abheben» Gewicht!

In einer besonders vorteilhaften Ausführungsform wird ein Stanzrohr zum Perikard geschleust. Dessen eines Ende ist in vorteilhafter Weiterführung zur Klinge geschliffen, so dass mit dieser ein Rundabschnitt aus dem Perikard in das Perikard gestanzt, werden kann. Das Stanzen weißt als Vorteil aus, eine saubere Stanz-, im Sinne von Trennkante auszubilden. Dieses verhindert ein weiteres Einreißen des Perikards.

[A7] Wenn während des Verfahrens ein Werkzeug durch die Vorrichtung in das Herz eingeführt wird, können an diesem in Abhängigkeit des Werkzeugs unterschiedliche Maßnahmen vorgenommen werden.

[A8] Wenn von der Vorrichtung während des Verfahrens ein Signal für die Fälle erzeugt wird, in denen sie den Herzbeutel oder das Herz berührt, trägt dessen Signalerzeugung positiv zur Schnelligkeit des Verfahrensdurchlaufs bei. Ein Signal an eine die Vorrichtung führende behandelnde Person ist geeignet, um die behandelnde Person zu einer Handlungsänderung, einem Bewegen des Außenteils über das Innenteil zu veranlassen. Das Signal bzw. eine Signalanzeige können automatisch, oder teilautomatisch, optisch, akustisch oder und haptisch ausgeführt sein. Wenn unterschiedliche Signale, z.B. ein akustisches Signal, wie ein Tönen und ein haptisches Signal, wie ein Rütteln oder Drücken kombiniert werden, erhöht sich die Signalwirkung, so das der Verfahrensablauf noch schneller und sicherer durchlaufen werden kann. Ein Kombinieren von Signalen erhöht die Redundanz. In Abhängigkeit des Signals kann auch ein automatischer Stop oder ein automatisches Einrasten einer Bremse vorgesehen sein. Dieses hat den Vorteil, dass eine Prozesspunkt angezeigt werden kann, ab dem mit erhöhter Sorgfalt vorzugehen ist. Von besonderem Vorteil ist, wenn ein automatischer Stop 1mm vor dem Perikard ausgelöst wird. Die Sicherheit des Verfahrensablauf wird weiter erhöht, wenn mindestens eine Kamera Teil des Verfahrens ist, mit der das Perikard betrachtet werden kann. Eine mechanische Bremse, z.B. eine Rast oder eine Federbremse bzw. eine elektronische Bremse z.B. eine Elektromagentbremse erhöht die Sicherheit weiter.

[A9] Wenn während des Verfahrens mittels mindestens einer Zacke an der Vorrichtung Perikard gegriffen wird, kann dieses durch leichtes Verschwenken der Vorrichtung oder deren Teilen hinter die Zacken verzwirbelt werden.

Durch ein Drehen oder Teildrehen, d.h. ein Verschenken der inneren Röhre gegenüber der äußeren Röhre werden deren Stirnflächen gegeneinander verschwenkt. Dieses Verschwenken resultiert vorteilhafterweise im Greifen von Perikard, wenn dieses vor bzw. an den Stirnflächen anliegt.

[A12] In einer bevorzugten Ausführungsform der Vorrichtung ist mindestens ein Innenteil vorhanden, wobei das Innenteil in der inneren Röhre beweglich angeordnet ist.

Wenn das Innenteil, z.B. ein Fühler oder eine Kanüle durch die innere Röhre schiebbar ist, kann das Innenteil sanft bis zum Perikard vorgeschoben werden, um dort seiner Funktion z.B. einem Fühlen, Greifen, Dichten oder Schließen zugeführt zu werden. Einer beweglichen Anordnung folgt somit der Vorteil einen Werkzeugwechsel zu ermöglichen. Im Beispiel des Schließens können Tacker-, Naht- und Hitze-, bzw. Laserwerkzeuge hintereinander verwendet werden, um geöffnetes Perikard wieder zu verschließen.

[A13] In einer bevorzugten Ausführungsform der Vorrichtung ist in mindestens einer Durchgangsbohrung des Innenteils mindestens eine Fühlersonde vorhanden ist.

Vorteilhafterweise meldet diese haptisch ein Signal, wann das Perikard erreicht ist. [A14] In einer bevorzugten Ausführungsform der Vorrichtung nach ist zwischen einer Fläche des Innenteils und einer Fläche des Fühlersondenkopfs mindestens ein Federelement angeordnet ist.

Dieses hat den Vorteil, ein Berühren des Fühlersonderkopf mit dem Perikard zu puffern.

[A15] Wenn mindestens eine Röhre flexibel, insbesondere ein Schlauch ist, ist die gesamte Vorrichtung flexibel. Dieses vereinfacht Ihre Handhabung.

### Figurenbeschreibung

Eine bevorzugte Ausführungsform der Erfindung wird beispielhaft anhand einer Zeichnung erläutert die Figuren der Zeichnung zeigen im Einzelnen:
Figur 1 eine erfindungsgemäße Vorrichtung in einer schematischen Schnittansicht,
Figur 1a eine erfindungsgemäße Vorrichtung in einer schematischen Schnittansicht von der Seite,
Figur 1b ein Außenteil der erfindungsgemäßen Vorrichtung in einer schematischen Schnittansicht von der Seite,
Figur 1c ein Innenteil der erfindungsgemäßen Vorrichtung in einer schematischen Schnittansicht von der Seite,
Figur 1d eine Detailansicht einer Sicherung,
Figur 1e eine Ansicht entlang des Schnittes E0-E0,
Figur 2 unterschiedliche Ansichten auf ein Außenteilende der erfindungsgemäßen Vorrichtung,
Figur 2a eine Detailansicht eines Außenteilendes in einer schematischen Schnittansicht von der Seite,
Figur 2b ein Außenteilende in einer Schnittansicht von rechts nach links,
Figur 2c ein Außenteilende in einer Schnittansicht von links nach rechts,
Figur 3 eine erste bevorzugte Weiterführung der erfindungsgemäßen Vorrichtung als sogenannter <<Zackengreifer>>,
Figur 3a eine schematische Ansicht auf eine erste Alternative des «Zackengreifer» von der Seite,
Figur 3b eine schematische Ansicht auf eine zweite Alternative des «Zackengreifer» von der Seite im schematischen Schnitt,
Figur 3c eine schematische Ansicht auf eine Weiterbildung des «Zackengreifers» von vorne,
Figur 4 eine zweite bevorzugte Weiterführung der erfindungsgemäßen Vorrichtung als sogenannter «Nutgreifer»,
Figur 4a eine schematische Ansicht auf die unterschiedlichen Alternativen des <<Nutgreifers>>,
Figur 5 einen schematischen Ablaufplan eines nicht erfindungsgemäßen Verfahrens,
Figur 6 eine schematische Darstellung von Lagen der erfindungsgemäßen Vorrichtung in ihrem Gebrauch,
Figur 6a eine Lage der erfindungsgemäßen Vorrichtung im menschlichen Körper,
Figur 6b eine Lage der erfindungsgemäßen Vorrichtung, in der eines ihrer Vorrichtungsenden das Perikard berührt,
Figur 6c eine Lage der erfindungsgemäßen Vorrichtung, in der eines ihrer Außenteilenden das berührt,
Figur 6d eine Lage der erfindungsgemäßen Vorrichtung, wobei das Perikard mit einem Außenteil Ende gegriffen wird,
Figur 6e eine alternative Ausführungsform einer erfindungsgemäßen Vorrichtung als Zackengreifer,
Figur 6f eine alternative Ausführungsform einer erfindungsgemäßen Vorrichtung als Zackengreifer in einer Einführungslage,
Figur 6g eine alternative Ausführungsform einer erfindungsgemäßen Vorrichtung als Zackengreifer in einer Grifflage.
Figur 7 eine erfindungsgemäße Vorrichtung in einer Gebrauchslage mit Dichtungen,
Figur 8 eine Detailansicht einer erfindungsgemäßen Vorrichtung,
Figur 8a eine Detailansicht einer erfindungsgemäßen Vorrichtung in einer gasdurchlässigen Gebrauchslage,
Figur 8b eine Detailansicht einer erfindungsgemäßen Vorrichtung in einer gasdichten Gebrauchslage,
Figur 9 eine Detailansicht einer erfindungsgemäßen Vorrichtung,
Figur 9a eine Detailansicht einer erfindungsgemäßen Vorrichtung in einer gasdurchlässigen Gebrauchslage,
Figur 9b eine Detailansicht einer erfindungsgemäßen Vorrichtung in einer gasdichten Gebrauchslage,
Figur 10 zeigt eine schematische Darstellung von Lagen einer erfindungsgemäßen Vorrichtung 10 zum Einbringen eines Implantates,
Figur 10a zeigt schematisch eine geöffnete Behandlungsstelle,
Figur 10b zeigt schematisch eine geöffnete Behandlungsstelle mit einem Soft-Tissue-Retraktor,
Figur 10c zeigt ein Spannen eines Soft-Tissue-Retraktor,
Figur 10d zeigt eine gedichtete Perikardöffnung.
Figur 11 zeigt eine schematische Darstellung von Lagen eines Implantats.
Figur 11a zeigt eine Lage eines Implantats innerhalb der erfindungsgemäßen Vorrichtung,
Figur 11b zeigt eine Lage eines Implantats innerhalb der erfindungsgemäßen Vorrichtung,
Figur 11c zeigt eine Lage eines Implantats,
Figur 11d zeigt ein Detail mit einem Implantatanker,
Figur 11e zeigt ein Detail mit einem Implantatanker.

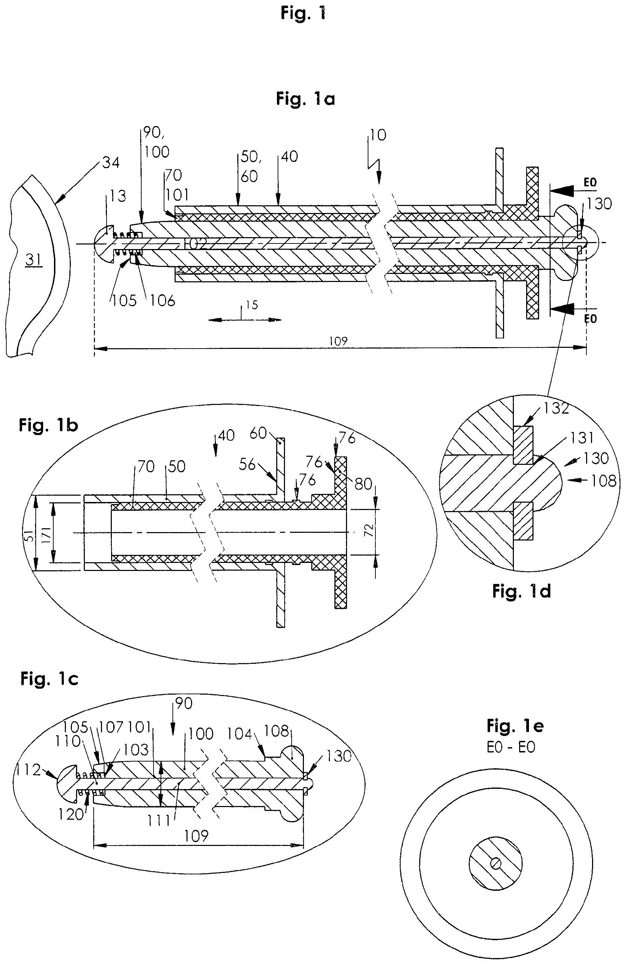

Figur 1 zeigt eine erfindungsgemäße Vorrichtung in einer schematischen Schnittansicht.

Phänomenologisch lehrt die erfindungsgemäße Vorrichtung ein Greifen von menschlichem Gewebe (nicht dargestellt) mittels eines Spannens eines Gewebeabschnitts (nicht dargestellt). Sie lehrt das Spannen dabei als Folge eines bedingten Verschiebens von mindestens zwei unterschiedlichen Gewebeunterabschnitten (nicht dargestellt). Die unterschiedlichen Gewebeunterabschnitte (nicht dargestellt) sind in unterschiedliche Richtungen zu verschieben, um sie somit untereinander zu verspannen. Dabei gilt, je stärker die Verschiebung, desto stärker die Spannung, desto stärker der Griff der erfindungsgemäßen Vorrichtung. Weil jedem Verschieben eines Gewebeunterabschnitts (nicht dargestellt) ein Aufbringen einer Kraft vorausgeht, kann die Lehre der erfindungsgemäßen Vorrichtung auch als Phänomen aufgefasst werden, dass ein Aufbringen mindestens zweier Kräfte in unterschiedlicher Richtung auf ein definiertes Flächenelement beschreibt.

Das Phänomen wird sinnlich als ein Greifen erfasst. Das Greifen ist ein Verspannen von Gewebe als Folge dessen multiaxialen Verschiebens. Dessen Unterphänomen, uniaxiales Verschieben von Gewebeabschnitten durch mindestens zwei Rotationen, wird vorliegend als<<Ringgreifen>> bezeichnet. Das Ringgreifen kann dabei ein Fixieren und Abziehen, ein Festhalten und Zurückziehen oder ein temporäres Fügen und Zwangsführen umfassen.

In Figur 1a ist die erfindungsgemäße Vorrichtung 10 in einer bevorzugten Ausführungsform als «Ringgreifer» dargestellt.

In einer bevorzugten Ausführungsform umfasst der «Ringgreifer» mindestens ein Außenteil 40, in dem ein Innenteil 90 geführt ist. Das Außenteil 40 umfasst wiederum eine äußere Röhre 50 und eine innere Röhre 70. Sowohl die äußere, als auch die innere Röhre 50, 70 sind in ihrem Querschnitt rund.

Die Röhre 50 und die innere Röhre 70 können in der bevorzugten Ausführungsform auch Schläuche (nicht dargestellt) oder Teile von Schläuchen (nicht dargestellt) sein, so dass ein Innenschlauch (nicht dargestellt) in einem Außenschlauch (nicht dargestellt) geführt ist. In der Ausführung als Schlauch ist die Bewegungsfreiheit für den Patienten erhöht. Als Schaft können die Schläuche eine flexible Welle aufweisen.

In dessen erster Unterausführungsform ist die äußere Röhre 50 als Äußeres Rändelrad 60, die innere Röhre 70 als inneres Rändelrad 80 gefertigt. Das Phänomen des Ringgreifens wird in dieser unter Ausführungsform erfassbar, weil sie mindestens zwei Teilfunktionalitäten miteinander verkettet: Kontaktieren des Außenteils 40 mit dem Perikard 34; Ringgreifen des kontaktierten Perikards 34. Ein (teil-) rotatorisches Verdrehen von des äußerem Rändelrad 60 gegen das innere Rändelrad - die Rotationsrichtung des äußeren Rändelrad (nicht dargestellt) zeigt entgegen der Rotationsrichtung des inneren Rändelrads (nicht dargestellt) - verspannt Gewebeabschnitte (nicht dargestellt) des Perikards 34 am Ort des Kontakts über dem Herz 31.

In dessen erster alternativen Ausführungsform weißt das Außenteil anstelle einer äußeren Röhre eine äußere Führung (nicht dargestellt), zum Beispiel eine äußere Linearführung (nicht dargestellt) auf. Präferierte Linearführung sind unter anderem Profilschienen mit einem U-, V- oder T Profil.

In dessen zweiter alternativer Ausführungsform umfasst das Außenteil sowohl eine äußere Röhre und eine innere Röhre oder eine Kombination beider in Ausgestaltung einer Polygonröhre (nicht dargestellt). Eine Polygonröhre ist in ihrem Querschnittsprofil mehreckig. Bevorzugte Querschnittsprofile für Polygonröhren sind Vier-, Fünf-, Sechs- oder Achtkantprofile.

In der bevorzugten Ausführungsform sind alle Baugruppen und Bauteile, insbesondere das äußere Rändelrad als auch das innere Rändelrad aus Aluminium oder Titan gefertigt. Vorzugsweise sind sie alle aus einem Aluminium-, oder Titanblock gefräst oder gedreht. In weiterführenden Alternativen können sie alle auch aus Stahl oder anderen biokompatiblen Materialien hergestellt werden. Präferiert sind biokompatible Materialien der ISO 10993, - neben Stahl insbesondere auch Kunststoff.

In der bevorzugten Ausführungsform ist in der äußeren Röhre 50 eine innere Röhre 70 gelagert. Der äußere Außenröhrendurchmesser (vgl. Figur 1b) entspricht dem äußeren Innenröhrendurchmesser (vgl. Figur 1b), so dass die Mantelfläche der inneren Röhre 70 zusammen mit einer Innenfläche der äußeren Röhre 50 die innere in der äußeren Röhre führt. In weiterführender Ausgestaltung führen Teilflächen die Röhren zueinander, sie können mindestens ein Mantelflächenabschnitt (nicht dargestellt) der inneren Röhre 70 oder mindestens ein Innenflächenabschnitt (nicht dargestellt) der äußeren Röhre 50 sein. In weiterführender Ausgestaltung ist die innere Röhre 70 in der äußeren Röhre 50 biaxial geführt. Die Achsen liegen in Längs-, und Umfangsrichtung des Ringgreifers 13, so dass die innere Röhre 70 gegenüber der äußeren Röhre 50 in zwei Freiheitsgrade manipulierbar ist.

In der bevorzugten Ausführungsform ist im Außenteil 40 ein Innenteil 90 unidirektional lose gelagert. In axialer Richtung 15 ist das Innenteil 90 im Außenteil 40 somit beweglich. Die Führung erzwingt ein Bewegen des Innenteil 90 im Außenteil 40 in axialer Richtung 15.

Die bevorzugte Ausführungsform in Figur 1a zeigt das Prinzip « zusammengesteckter Röhren». Der Ringgreifer 13 wird gebildet, indem dessen Innenteil 90 in das Außenteil 40 gesteckt ist. Das Außenteil wird gebildet, indem die innere Röhre 70 in die äußere Röhre 50 gesteckt ist. Damit gilt der Satz, «alle Bauteile sind zusammengesteckt>>. Weil auch Innenteil 90 und Außenteil 40 der Art nach Röhren sind, gilt der Satz, <<alle Bauteile sind Röhren>>. Damit konkludiert im Modus Podens, <<alle Röhren sind zusammengesteckt>>. Dieses Prinzip kann auch als «ineinandergesteckte Röhren» bezeichnet werden.

In dessen zweiter Unterausführungsform ist das Innenteil 90 als Stift 100 ausgeführt. In den Stift 100 ist mindestens eine Durchgangsbohrung 102 gefertigt. Sie ist in bevorzugter Weise über dessen ganze Stiftlänge 109 gebohrt. In die Stiftspitze 105 ist ein Stiftloch 106 gesenkt. Der Stift 100 weist einen äußeren Stiftdurchmesser 101 auf.

In der bevorzugten Ausführungsform umfasst das Innenteil 90, einen Fühler. Dieser zergliedert sich in einen Fühlerschaft (vgl. Figur 1c) und einen Fühlerkopf (vgl. Figur 1c). Der Fühlerschaft (vgl. Figur 1c) ist in die Durchgangsbohrung (vgl. Figur 1c) des Innenteils 90 gesteckt. Mittels einer Sicherung 130 ist er auf der der Innenteilspitze entgegengesetzten Seite des Ringgreifers 13 gegen ein Herausrutschen in axialer Richtung 15 in Richtung der Vorrichtungsspitze 13 gesichert.

Figur 1b zeigt ein Außenteil 40 der erfindungsgemäßen Vorrichtung in einer schematischen Schnittansicht von der Seite.

In der bevorzugten Ausführungsform ist die innere Röhre 70 in die äußere Röhre 50 gesteckt, ein inneres Rändelrad 60 steckt in einem äußeren Rändelrad 80 Die innere Röhre 70 weist mindestens drei Innenröhrenbünde 76 auf: - von rechts nach linkseinen ersten Innenröhrenbund 76 am handseitigen Vorrichtungsende 12; einen zweiten Innenröhrenbund 76 mit Abstand zum ersten; einen dritten Innenröhrenbund 76 mit Abstand zum zweiten Innenröhrenbund 76. Der erste Innenröhrenbund 76 ist ein Handgriff, der zweite Innenröhrenbund 76 sichert ein Herausrutschen der inneren Röhre 70 aus der äußeren Röhre 50 in axialer Richtung 15 in Richtung der Vorrichtungsspitze 13, der dritte Innenröhrenbund 76 kann die innere Röhre 70 gegen ein vollständig gegen axiales Bewegen sichern. Dazu wird der dritte Innenröhrenbund 76 in einer Nut der äußeren Röhre geführt.

Auch in die äußere Röhre 50 ist mindestens ein erster Außenröhrenbund 56 gefertigt, insbesondere gedreht. Auch dieser Außenröhrenbund 56 bildet einen Handgriff aus.

In allen Ausführungen können alle Handgriffe gerändelt sein, so dass eine behandelnde Person diese gut festhalten kann. Ferner kann in allen Ausführungen der äußere Außenröhrendurchmesser 51 des Handriffs der äußeren Röhre 50 größer sein als der äußere Durchmesser der Innenröhre 71 des, des inneren Handgriffs. Noch ferner können in allen Ausführungen der äußere Durchmesser als auch der innere Durchmesser entlang der Vorrichtungslänge variieren.

Der innere Innenröhrendurchmesser 72 ist derart gefertigt, dass eine Vielzahl an Werkzeugen durch ihn führbar ist.

Figur 1c zeigt ein Innenteil 90 der erfindungsgemäßen Vorrichtung 10 in einer schematischen Schnittansicht von der Seite.

Das Innenteil 90 umfasst in der bevorzugten Ausführungsform mindestens vier Bauteile: einen Stift 100, einen Fühler 110, ein Federelement 120 und eine Sicherung 130.

Der Stift 100 ist in bevorzugte Ausführungsform gestuft gefertigt. Die Stiftspitze 105, sie zeigt in Richtung des Perikards (nicht dargestellt), ist konisch. Von der Stiftspitze 105 nimmt der äußere Stiftdurchmesser 101 in Richtung Stiftknauf 108 zu. Am äußersten Ende der Stiftspitze 105 ist der Stiftdurchmesser 101 somit am Geringsten.

In die Stiftspitze 105 ist ein Stiftlock gesenkt. Dessen Stiftlochdurchmesser 107 ist größer als der Durchgangsbohrungsdurchmesser 103. An seinem einen Ende weißt der Stift 100 einen Stiftbund 104 auf.

Der Fühler 110 umfasst einen Fühlerschaft 111 und einen Fühlerkopf 112.

In der bevorzugten Ausführungsform wird das Innenteil 90 montiert, in dem im ersten Schritt das Federelement 120 über den Fühlerschaft 112 gesteckt wird, um anschließend im zweiten Schritt den Fühlerschaft mit Federelement 120 durch die Durchgangsbohrung des Stifts 100 zu stecken, so dass abschließend im dritten Schritt der Fühler 110 gegen ein Herausfallen-, oder Rutschen aus dem Stift 100 mittels der Sicherung 130 (vgl. Figur 1d) gesichert werden kann.

Figur 1d zeigt eine Detailansicht einer Sicherung. Die Sicherung befindet sich am freien Ende des Stiftknauf 108. Das freie Ende des Stiftknauf 108 liegt in einer bevorzugten Gebrauchslage ventral. In einer alternativen Gebrauchslage liegt es lateral. Beide Lagen des freien Endes des Stiftknauf 108 kennzeichnen dessen Lage gemäß der anatomischen Hauptrichtungen vom Körper weg zeigend. Die Sicherung 130 verhindert ein Herausrutschen des Fühlers in dorsale Richtung aus dem Stift, in dem ein Sicherungsring 132 in eine Sicherungsnut 131 passt, vorzugsweise presspasst.

In einer alternativen Ausführungsform kann auch ein Sicherungssplint (nicht dargestellt) in eine Sicherungsbohrung (nicht dargestellt), die quer zur Seele des Stifts gebohrt ist, form- oder reibschlüssig, z.B. gesteckt, gesteckt und gebogen, gepasst, insbesondere pressgepasst werden.

Figur 1e zeigt eine Ansicht entlang des Schnittes EO-EO aus Figur 1a. Im Schnitt werden die Größenrelationen der oben besprochenen Handgriffe deutlich.

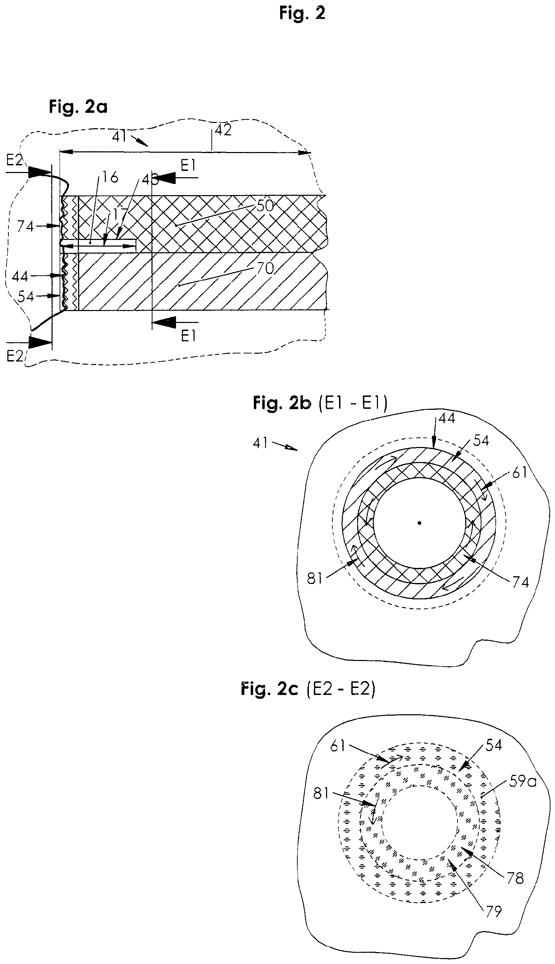

Figur 2 zeigt unterschiedliche Ansichten auf ein Außenteilende der erfindungsgemäßen Vorrichtung. Das Außenteilende beschreibt einen dorsalen Abschnitt des Außenteils.

Figur 2a zeigt eine Detailansicht eines Außenteilendes 41 in einer schematischen Schnittansicht von der Seite.

In bevorzugter Ausführungsform ist seine Außenteilendlänge 42 ist ca. 2 cm lang, in weiterer Ausgestaltung beträgt sie 1 cm. In alternativen Ausführungsformen ist sie entsprechend der Elastizität des Perikards (nicht dargestellt) oder anderer innerer Häute (nicht dargestellt), Körperschichten (nicht dargestellt), Organe oder der Außenhaut ausgelegt. An der dorsalen Spitze des Außenteilendes befindet sich zwischen der äußeren Röhre 50 und der inneren Röhre 70 ein Volumen 16.

In bevorzugter Ausführungsform ist das Volumen 16 Folge eines aus der äußeren Röhre 50 herausgefertigten Außenteilendabsatz 43. Dieser ist entlang des inneren Umfanges des Außenteilendes 41 gefertigt, insbesondere gedreht oder gefräst.

In einer alternativen Ausführungsform kann auch in die innere Röhre 70 ein Absatz (nicht dargestellt) oder Absätze in die äußere Röhre 50 und innere Röhre 70 gefertigt werden.

In Weitergestaltung sowohl bevorzugter oder alternativer Ausführungen können die Absätze unterschiedliche Profile, zum Beispiel geriffelte, steigende, geschwungene, gezackte oder verjüngende Profile (nicht dargestellt) aufweisen. Auch können eine Mehrzahl von Außenteilendabsätzen 43 entlang des inneren Umfangs des Außenteilendes 41 in die äußere Röhre 50 oder die innere Röhre 70 gefertigt werden.

Insbesondere die Innenröhrenenden 74, Außenteilendfläche 44 und Außenröhrenendfläche 54, können einzeln oder kombiniert profiliert sein. Unterschiedliche Profile erhöhen die Reibkräfte entlang der Flächen und Enden.

Figur 2b zeigt ein Außenteilende in einer Detailansicht, als eine Schnittansicht von rechts nach links. Zu sehen ist die Außenteilendfläche 44 des Außenteilenden 41. Die Detailansicht offenbart, dass die Außenteilendfläche 44 aus einer Außenröhrenendfläche 54 und eine Innenröhrenendfläche 74 gebildet ist.

In einer bevorzugten Ausführungsform beschreibt diese eine Gesamtfläche. Die Außenteilendfläche 44 liegt in Richtung dorsal, d.h. ihr Flächenvektor (nicht dargestellt) zeigt gemäß den anatomischen Hauptrichtungen in Richtung des menschlichen Körpers (nicht dargestellt), gleiches gilt für die Flächenvektoren (nicht dargestellt) ihrer Teilflächen.

In weiterführender Ausgestaltung können sowohl die Außenteilendfläche 44, das ist eine Stirnfläche als auch deren Teilflächen oder Abschnitte profiliert sein. Vorzugsweise ist das Profil so gefertigt, dass sich eine hohe Rauigkeit ergibt. Exemplarisch gilt, dass eine hohe Rauigkeit dann gegeben ist, wenn das Profil einen in den Ingenieurswissenschaften definierte Rauhigkeitswert aufweist, dessen hoher oder niedriger Wert per Definition das Profil als rau auszeichnet. Als ein Rauhigkeitswert - neben vielen anderen - kann der sogenannte RA-Wert herangezogen werden.

In weiterführender Ausgestaltung differieren Rauigkeiten, um im Beispiel zu bleiben der RA-Werte, der Außenröhrenendfläche 54 und der Innenröhrenendfläche 74.

In der Gebrauchslage ist eine Rotationsrichtung des äußeren Rändelrads 61 entgegengesetzt zur Rotationsrichtung des inneren Rändelrad 81.

Figur 2c zeigt ein Außenteilende als Detailansicht, im Gegensatz zu Figur 2b aber als Schnittansicht von links nach rechts.

In die Außenröhrenendfläche 54 ist ein Außenröhrenendflächenprofil eingearbeitet, insbesondere ein Profil, dass Außenröhrenendflächenkerben 59a und Innenröhrenendflächenkerben 79 umfasst. Die Außenröhrenendflächenkerben 59a sind in bevorzugte Ausführungsform radial in die Außenröhrenendfläche 54 in gleichem Abstand (nicht dargestellt) zueinander gefertigt. Auch die Innenröhrenendflächenkerben sind radial als Innenröhrenendflächenprofil 78 in die Innenröhrenendfläche in gleichem Abstand (nicht dargestellt) zueinander gefertigt. Die Hauptrichtung der Außenröhrenendflächenkerben 59a verlaufen im Wesentlichen entgegengesetzt, schräg, zu der Hauptrichtung der Innenröhrenendflächenkerben 79.

In weiterführender Ausgestaltung differieren das Außenröhrenendflächenprofil und das Innenröhrenendflächenprofil 78. Diese Differenz ist dadurch gekennzeichnet, dass mindestens eine erste Hauptrichtung (nicht dargestellt) des Außenröhrenendflächenprofils entgegengesetzt zu mindestens einer zweiten Hauptrichtung des Innenröhrenendflächenprofils 78 zeigt.

Alternativ können in weiterführenden Ausgestaltungen die erste und zweite Hauptrichtung im Wesentlichen entgegengesetzt sein. Wenn die erste und die zweite Hauptrichtung vektoriell interpretiert werden; und wenn ein erster Teilvektor des ersten Hauptrichtungsvektors und ein zweiter Teilvektor des zweiten Hauptrichtungssektors parallel aber entgegengesetzt verlaufen, kann indiziert sein, dass die erste Hauptrichtung und die zweite Hauptrichtung im Wesentlichen entgegengesetzt verlaufen.

In weiterführender Ausgestaltung können die erste und zweite Hauptrichtung beliebig zueinander liegen.

Wenn die Rotationsrichtung des äußeren Rändelrads 61 entgegengesetzt zur Rotationsrichtung des inneren Rändelrad 81 ist, haken die Profile ineinander.

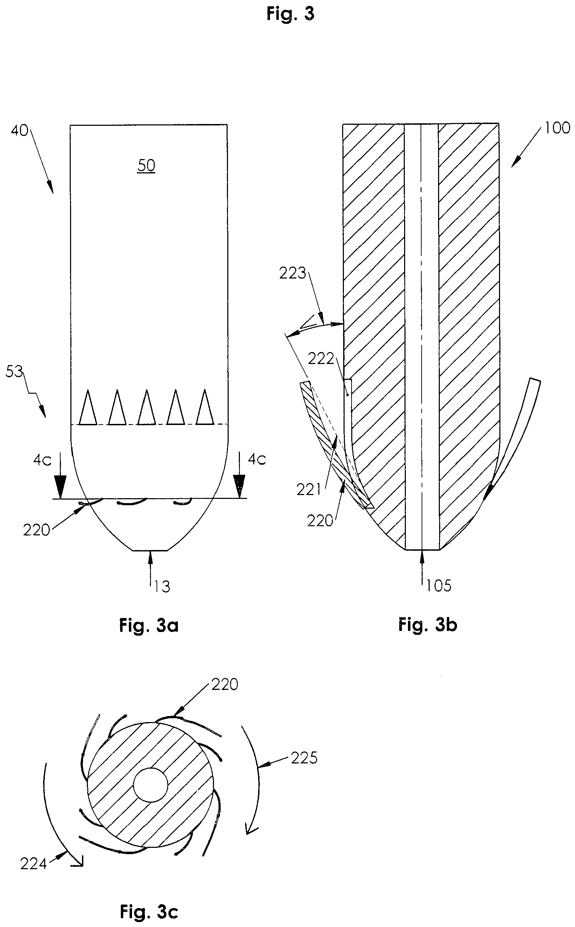

Figur 3 zeigt eine bevorzugte Weiterführung der erfindungsgemäßen Vorrichtung.

Sie wird «Zackengreifer» genannt. Der « Zackengreifer» versinnlicht das Phänomen des mechanischen Perikardabhubs vom Herz mittels eines Hintergreifens von Perikard durch Zacken. Dieses Hintergreifen kann auch als Hinter-ZackenGreifen bezeichnet werden. Alternative Bezeichnungen für den <<Zackengreifer>> sind <<Hinterzackengreifer>> oder <<Schuppengreifer>>.

Eine Zacke beschreibt in der Regel mindestens eine Spitze, einen Haken, einen Widerhaken, eine Lamelle oder eine Schuppe am Rande. An diesen Rand, einem Ende, ist sie in der Regel an einem ihrer Zackenenden befestigt. An mindestens einem anderen Zackenende ist die Zacke scharfkantig, insbesondere angeschliffen und angefeilt. Eine Zacke kann auch als Zahn, zum Beispiel Sägeblattzahne aufgefasst werden.

Figur 3a zeigt eine schematische Ansicht auf die erste alternative Ausführungsform.

Sie zeigt einen <<Zackengreifer>> als <<Außenteilzackengreifer>>.

In einer bevorzugten Ausführungsform sind die Zacken 220 an die Vorrichtungsspitze 13 am Außenteil 40, in weiterer Ausführung im Bereich des Außenteilendes oder im Bereich der Innenteilspitze, vorzugsweise im Bereich der Stiftspitze angeordnet. Für den Fall, dass die Zacken 220 im Bereich des Außenteilendes angeordnet sind, ist in noch weiterer Ausführung ein Anordnen von mindestens einem Zacken 220 entweder im Bereich des Außenröhrenendes und/oder im Bereich des Innenröhrenendes des bevorzugt.

In bevorzugter Ausführungsform sind dazu am Außenröhrenende 53 Zacken 220 vorgesehen. In weiterführender Ausgestaltung können diese einstückig mit der Außenröhre 50 gefertigt sein. In präferierter Ausgestaltung sind die Zacken 220 mit der Außenröhre 50 in einem Schuss spritzgegossen. Alternativ können diese als einzelne Lamellen auf das Außenröhrenende 53 aufgeschweißt oder aufgelötet sein.

Figur 3b zeigt eine schematische Ansicht auf die erste alternative Ausführungsform, mit einem Stift 100 zum «zackengreifen».

In bevorzugter Ausführung sind die Zacken 220 dabei in eine Zackentasche 222, in alternativer Ausführung auf die äußere Mantelfläche des Stifts 100, einstückig mit dem Stift 100, vorzugsweise an der Stiftspitze 105 im Spritzgussverfahren, gegossen.

In bevorzugter Ausführungsform liegen alle Zacken 220 in gleichem Anstellwinkel 223 zum Stift 100, z.B. Zackenmittellinie 221 zu Stiftachse.

Figur 3c zeigt eine schematische Ansicht auf die erste alternative Ausführungsform von vorne. Zacken 220 sind an die Stiftspitze als Schuppen - Zacken 220 in geschuppter Form - entweder geklebt oder geschweißt. Die Zackenlänge (nicht dargestellt) der Zacken 220 variiert. Daher überlappen sich die Zacken 220 in Teilen. In Richtung der Zackenspitzen, d.h. im Uhrzeigersinn drehen bzw. schwenken die Zacken 220 im Greifrichtung 225, hingegen laufen sie entgegen dem Uhrzeigersinn im Freilauf 224. Ein Verschwenken des Zackengreifers umfasst kleine, d.h. graduelles Drehen des Zackengreifers um seine Achse. Das Verschwenken kann sowohl in Greifrichtung 225 als auch in Freilaufrichtung 224 erfolgen.

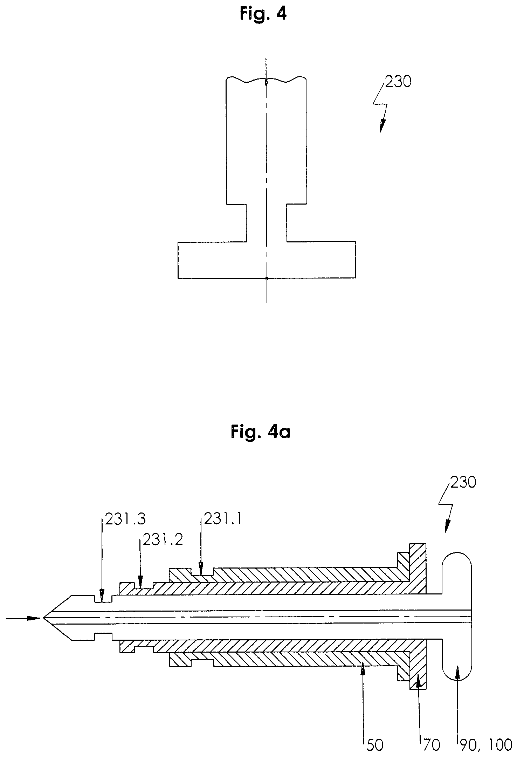

Figur 4 zeigt eine schematische Ansicht auf eine weitere alternative Ausführungsform.

Diese Ausführungsform kann als «Nutgreifer» 230 bezeichnet werden.

Der « Nutgreifer» versinnlicht das Phänomen eines mechanischen Perikardhubs, indem in eine in ihm gefertigte Nut Perikard fließt, gezogen, oder gedrückt werden kann, so dass dieses dort verklemmt werden kann.

Figur 4a zeigt eine erste Weiterbildung des Nutgreifer 230.

In erster bevorzugter Ausführung, dem <<Außenröhrennutgreifer>> ist dieser dadurch gekennzeichnet, dass mindestens eine Nut 231.1 in die äußere Röhre 50 gefertigt ist.

In zweiter bevorzugter Ausführungsform, dem <<Innenröhrennutgreifer>> ist dieser dadurch gekennzeichnet, dass mindestens eine Nut 231.2 in die innere Röhre 70, insbesondere im Bereich der Vorrichtungsspitze gefertigt ist.

In dritter bevorzugter Ausführungsform, dem <<Innenteilnutgreifer>> ist dieser dadurch gekennzeichnet, dass mindestens eine Nut 231.3 in das Innenteil 90, vorzugsweise in den Stift 100, insbesondere im Bereich der Stiftspitze gefertigt ist. Das präferierte Fertigungsverfahren zum Einbringen von mindestens einer Nut in eine der vorgeschlagenen Weiterbildungen des Nutgreifer ist Spritzgießen. Die Nut wird also während der Fertigung der jeweiligen Weiterbildung eingearbeitet. z.B. gefräst. Alternativ kann die Nut nach Fertigstellung der Weiterbildung in einem späteren Arbeitsschritt hergestellt oder nachgearbeitet werden. Bevorzugter Weise ist sie dazu ausgefräst.

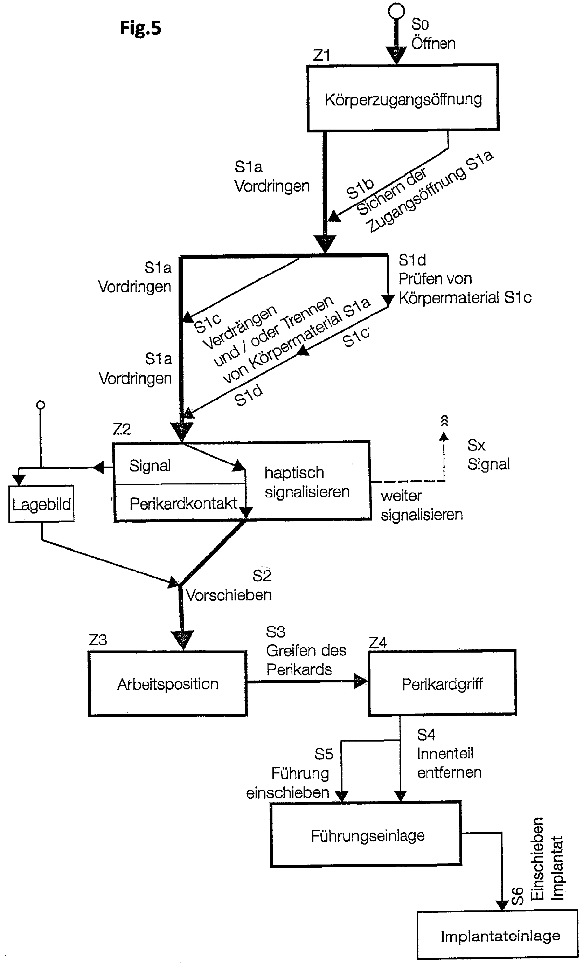

Im Gebrauch wird eine Weiterbildung der zweiten Ausführungsform soweit in einen Gewebeabschnitt oder einen Gewebeunterabschnitt, wie zum Beispiel das Perikard gedrückt, bis dieses in oder hinter die Nut fließt und oder gedrückt wird. Durch ein Verschwenken, Rotieren oder Teil rotieren, insbesondere Drehen des Nutgreifer wird sodann das Perikard verdreht, also verzwirbelt, sodass das Perikard reib- und/oder formschlüssig in der Nut gelagert, also fixiert, geklemmt, im Sinne von gegriffen ist. Figur 5 zeigt einen schematischen Ablaufplan eines Verfahrens. Der bevorzugte Ablauf wird in Figur 5 durch Schritte dargestellt. Sie sind mit « S » bezeichnet. Dicke Pfeile kennzeichnen Hauptschritte, dünne Pfeile kennzeichnen alternative Zwischenschritte. Dicke, wie Dünne laufen nach Plan, allerdings Dünne meist in Schleifen, um Dicke nicht zu überholen. Im Gegensatz zu Schritten sind Zustände als Boxen - Kisten- dargestellt. Sie sind mit « Z » bezeichnet.

In der bevorzugten Ausführungsform In der Gebrauchslage zeigen die Flächenvektoren (nicht dargestellt) sowohl der Außenröhrenendfläche und der Innenröhrenendfläche in Richtung des Perikards.

In einem bevorzugten Ausführungsschritt SO ist ein menschlicher Körper (nicht dargestellt) zu eröffnen. Dazu wird dieser mit einem Skalpell aufgeschnitten, um eine Körperzugangsöffnung zu schaffen. Deren Ausdehnung kann dabei zwischen mindestens zwei Extrempositionen variieren: einer minimal möglichen Körperzugangsöffnung, z.B. einer Punktation und einer maximal möglichen Körperzugangsöffnung, z.B. einem geöffneten Brustkorb.

Bevorzugter Weise wird eine behandelnde Person einen subxiphoidalen Körperzugang schaffen. Alternativ kann ein Zugang interkostal oder apikal eröffnet werden. Die vorgenannten Begriffe subxiphoidal, interkostal und apikal beschreiben anatomische Hauptrichtungen.

Nach dem eine Körperzugangs geschaffen wurde, kann durch diese eine Vorrichtung, insbesondere eine erfindungsgemäße Vorrichtung, ein Ring-, Zacken- oder Nutgreifer (nicht dargestellt) in den Patienten (nicht dargestellt) eingeführt werden.

In einem alternativen Zwischenschritt S1b kann die Körperzugangsöffnung mittels eines geeigneten Werkzeuges (nicht dargestellt) von der behandelnden Person gesichert werden.

Mit der erfindungsgemäßen Vorrichtung wird anschließend im Schritt S1a durch den menschlichen Körper vorgedrungen, in dem eine behandelnde Person diesen vorschiebt. Das Vorschieben ist dabei von einem Sondieren, einem vorsichtigen Erkunden als auch von einem Dilatieren, einem Raum schaffen gekennzeichnet.

Dabei wird in mindestens einem Zwischenschritt S1c umliegendes menschliches Gewebe (nicht dargestellt), Organe (nicht dargestellt) oder Häute (nicht dargestellt) gegebenenfalls parallel verdrängt oder durchtrennt, um die erfindungsgemäße Vorrichtung weiter durch den menschlichen Körper (nicht dargestellt) zu treiben.

In einem alternativen Zwischenschritt S1d kann die behandelnde Person prüfen, welches menschliche Gewebe (nicht dargestellt) sie verdrängt oder durchtrennt. Sie kann ferner prüfen, ob sie die erfindungsgemäße Vorrichtung noch in Richtung des Perikards treibt.

Sobald die erfindungsgemäße Vorrichtung mit einem ihrer Vorrichtungsenden Perikard berührt, erzeugt dieser ein Signal. In einer bevorzugten Ausführungsform ist das Signal haptisch, das Signal wird also von der behandelnden Person gefühlt. Die Berührung wird dabei durch spürbar wachsenden Widerstand erfahren. In einer zweiten bevorzugten Ausführungsform wird das Signal durch einen elektrischen Signalgeber erzeugt, alternativ z.B. kann dieser Signale akustisch oder optisch erzeugen. Die Signale werden dabei vom Signalgeber aufgezeichnet, so dass eine Behandlung nachvollzogen werden kann.

Wenn der Zustand Z2 erreicht ist, können Zwischenschritte von der behandelnden Person eingeleitet werden.

Bevorzugte Zwischenschritte dienen einer Aufnahme eines Lagebilds. Aufzunehmen ist ein Röntgen- oder Ultraschallbilds oder eines Elektrokardiogramms (EKG) oder einer Kombination daraus.

Als nächstes wird von der behandelnden Person das Außenteil über das Innenteil geschoben, bis die Innenteilspitze das Perikard berührt. Liegt dieser Zustand «Z3» vor, ist die erfindungsgemäße Vorrichtung in Arbeitsposition.

Wenn die erfindungsgemäße Vorrichtung in Arbeitsposition ist und ein «Ringgreifer» ist, wird das Perikard im Wege eines Insich- Verdrehens des Ringgreifers gegriffen. Durch das Rotieren oder Teilrotieren der äußeren Röhre und der inneren Röhre wird das Perikard gegriffen. Das Rotieren oder Teilrotieren der vorgenannten Röhren erfolgt bevorzugter Weise in entgegengesetzter Richtung. In weiterführender Ausgestaltung steht also die Rotationsrichtung des inneren Rändelrads der Rotation Richtung des äußeren Rändelrad entgegen.

Wenn die erfindungsgemäße Vorrichtung in Arbeitsposition ist und als Zackengreifer ausgeführt ist, wird das Perikard (nicht dargestellt) im Wege eines Verhakens des Perikards (nicht dargestellt) gegriffen. Die auf ihm befindlichen Zacken (nicht dargestellt) werden in das Perikard eingedrückt, vorzugsweise leicht eingedrückt und der Zackenreifer leicht rotiert oder oszilliert, so das Perikard (nicht dargestellt) oder Perikardabschnitte (nicht dargestellt) hinter die Zacken (nicht dargestellt) fließt, gedrückt, geschoben oder gefördert wird und dort verhakt.

Wenn die erfindungsgemäße Vorrichtung in Arbeitsposition ist und als Nutgreifer ausgeführt ist, wird das Perikard im Wege eines Verdrillens des selbigen gegriffen. Der Nutgreifer wird leicht in das Perikard eingedrückt und/oder dabei rotiert oder oszilliert, so das Perikard oder dessen Perikardabschnitte (nicht dargestellt) in die Nut fließen, gedrückt oder gefördert werden. Durchzunehmende Rotation des Nutgreifer wird das Perikard weiter in die Nut gefördert, in dem sie hineingezogen wird. Die Zunahme an Perikard komprimiert dieses in der Nut , in deren Folge sein Reib- und Formschluss zunimmt.

Wenn die behandelnde Person das Perikard im Verfahrensschritt S3 gegriffen hat und gegebenenfalls vom Griff überzeugt ist, dann liegt der Zustand «Z4»vor, der hier genannte Perikardgriff.

In weiterer Ausgestaltung eines Verfahrens kann nun das Innenteil aus dem Außenteil entfernt werden, Verfahrensschritt S4, so dass ein Zugang zum Perikard als Tunnel, Durchlass, Schlauch oder Röhre durch die erfindungsgemäße Vorrichtung , mindestens einen Vorrichtungstunnel eröffnet ist.

Durch den Zugang kann ein Stanzrohr (nicht dargestellt) eingeführt werden, um eine Punktion in das Perikard zu stanzen. In bevorzugter Ausführungsform ist das Stanzrohr eine Kanüle.

In weiterer Ausgestaltung eines Verfahrens kann die behandelnde Person durch den Vorrichtungstunnel ein Implantat in das Perikard einführen. Vorzugsweise ist das Implantat eine Drainage, eine Sonde oder Kissen.

Alternativ und oder konsekutiven kann das Implantat durch eine Implantatführung in oder aus dem Perikard geführt werden.

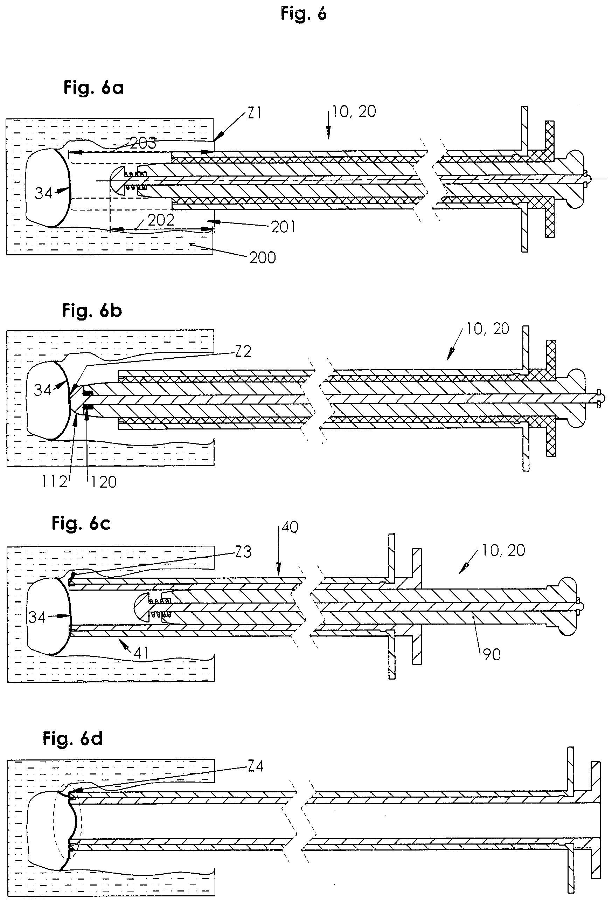

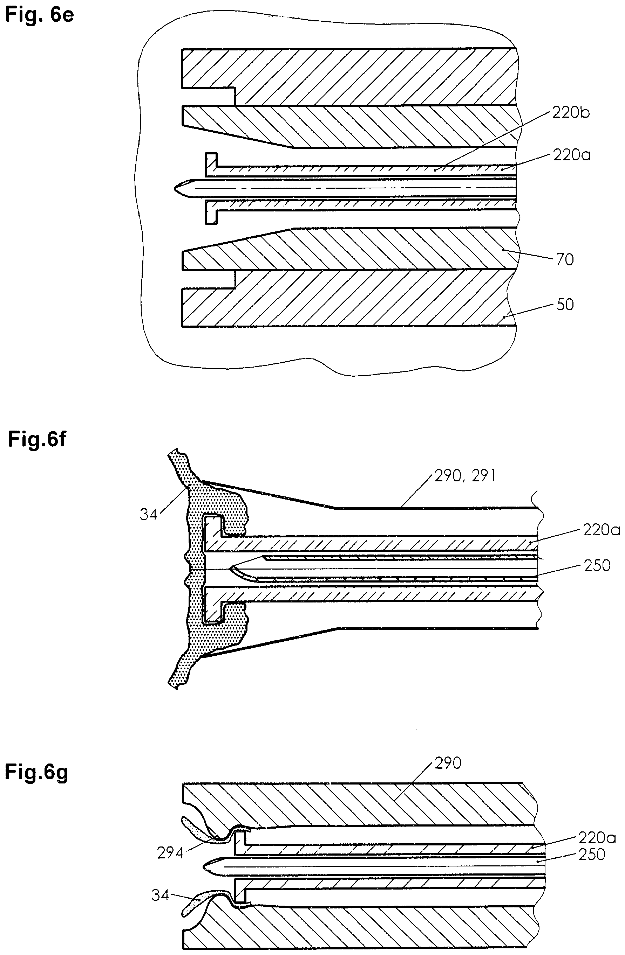

Figur 6 zeigt eine schematische Darstellung von Lagen der erfindungsgemäßen Vorrichtung in ihrem Gebrauch.

Figur 6a zeigt eine Lage der erfindungsgemäßen Vorrichtung 10. Die erfindungsgemäße Vorrichtung 10 ist als Ringgreifer 20 ausgeführt und in den menschlichen Körper 200 durch die Körperzugangsöffnung 201 eingeführt. Die Vorrichtungsspitze zeigt eine Eindringtiefe 202, ihren Abstand zur Körperzugangsöffnung 201.

Die Arbeitstiefe 203 beschreibt den Abstand zwischen Perikard 34 und Körperzugangsöffnung 201.Der Ringgreifer 20 ist in Arbeitsposition «Z4», wenn die Vorrichtungsspitze am Perikard 34 anliegt, also es berührt.

Figur 6b zeigt eine Lage der erfindungsgemäßen Vorrichtung 10 in Ausführung als Ringgreifer 20 im Perikardkontakt, «Z2».

In der Arbeitslage drückt der Fühlerkopf 112 gegen das Perikard 34. Weil das Perikard 34 durch den Schlag des Herzens (nicht dargestellt) pulsiert, pulsiert auch der Fühler, seine Bewegung folgt konsekutiven aus der Stärke der Perikardpulsation und der Stärke des Federelements 120.

Im bevorzugte Ausführungsform ist das Federelement 120 eine Spiral- oder Tellerfeder, ein Schaumstück oder ein Gummistück.

Figur 6c zeigt eine Arbeitsposition «Z3» der erfindungsgemäßen Vorrichtung 10 in Ausführung als Ringgreifer 20.

Das Außenteil 40 berührt mit seinem Außenteilende 41 das Perikard 34. Das Innenteil 90 ist vom Perikard 34 beabstandet.

Figur 6d zeigt eine erfindungsgemäße Vorrichtung 10 mit gegriffenem Perikard, Perikardgriff <<Z4>>.

Figur 6e zeigt eine alternative Ausführungsform einer erfindungsgemäßen Vorrichtung mit Zackengreifer 220a in einer Einführungslage. Der Zackengreifer 220a wird dabei durch eine innere Röhre 70 und eine äußere Röhre 50 an seinen Zackengreiferschaft 220b geführt.

Figur 6f zeigt eine weitere bevorzugte Variante. Der Zackengreifer 220a ist lediglich durch eine Stützstruktur 290 geführt. Diese kann eine innere Röhre (nicht dargestellt), ein Schlauch (nicht dargestellt) oder ein sogenannter Soft-Tissue-Retraktor 291 sein. Wenn die Stützstruktur 290 ein Soft-Tissue-Retraktor 291 ist, ist dieser auf mindestens einem Abschnitt von Perikard 34 positionierbar. In bevorzugter Weiterbildung weißt der Soft-Tissue-Retraktor 291 an seinem apikalen Ende einen Trichter auf. Durch ihn ist der Zackengreifer 220a vorschiebbar bis er das Perikard 34 berührt. Durch sein leichtes Verschwenken im Perikard 34 wird in dieses durch die Zacken 220 (vgl. Figur 3) ein Sackloch hineingeschnitten, in das der Zackengreifer 220 gesteckt werden kann. Ist dadurch das Perikard 34 gegriffen, ist eine Punktionsnadel 250 vorschiebbar um das Perikard 34 zu öffnen.

In Figur 6f ist der Zackengreifer 220a in einer Grifflage, d.h. der Zackengreifer 220a und das Perikard 34 bilden zu mindestens einen temporären Halt. In bevorzugter Ausführungsform resultiert dieser aus einem Umfließen der Zacken (vgl. Fig.3c) mit Perikard 34 bzw. Perikardanteilen (vgl. Figur 3c) bzw. aus einem mechanischen Eindringen der Zacken 220 in der Perikard 34.

Figur 6g zeigt eine weitere bevorzugte Ausführungsform. Diese weist mindestens einen Feststellabsatz 294 auf. Im Gebrauch wird der Zackengreifer 220a durch eine Stützstruktur 290 vorgeschoben und Perikard 34 wird von Zacken (nicht dargestellt, vgl. Figur 3 gegriffen). Das gegriffene Perikard 34 wird mitgenommen und hinter den Feststellabsatz 294 gezogen, um es dadurch festzuklemmen. Phänomenologisch bilden die Zacken (nicht dargestellt) somit Mitnehmer aus. Ist das Perikard 34 festgeklemmt, wird ist eine Punktionsnadel 250 vorschiebbar, um das Perikard 34 zu öffnen. Die Stützstruktur 290 ist aus einem elastischen Material geformt, so dass dieses sich Aufbiegen kann, wenn Perikard 34 durch den Zackengreifer 220a hinter den Feststellabsatz 294 gezogen wird.

Unabhängig von der Art der Ausführungsvariante sind am Zackengreifer an seinem apikalen Ende umlaufende Zacken (vgl. Figur 3) ausgebildet, d.h. mindestens zwei Zacken sind entlang des Umfangs am Zackengreifer angeordnet Diese sind aus dem vollen Titan oder Aluminium gefräst. Bevorzugterweise ist der Zackengreifer dafür aus einem Stück ausgeführt. Alternativ kann der Zackengreifer auch zweistückig ausgeführt sein. Dazu ist dessen apikales Ende aus einem Rohling gestanzt, so dass ein Kreissägenblatt, bzw. ein Fräser (vgl. Figur 3c) resultiert. Dieses ist alternativ an mindestens einen Abschnitt des Zackengreiferschaft geklebt, geklemmt, gepresst, oder geschweißt.

In einer bevorzugten Ausführungsform läuft der Zackengreifer entgegen der Verlaufsform der Zacken frei. Diese Richtung bildet den sogenannten Freilauf aus. Entgegengesetzt, d.h. im Sinne des Uhrzeigerlaufs zeigt Figur c einen Lauf des Zackengreifers in Greifrichtung. Das am Umfang mit Zacken versehene Greifwerkzeug kann sich während der Bewegung in der Umfangsrichtung mit den Zacken in die Wandung des Sacklochs des Perikards eingraben und in der anderen Umfangsrichtung an der Wand des Perikards entlang gleiten.

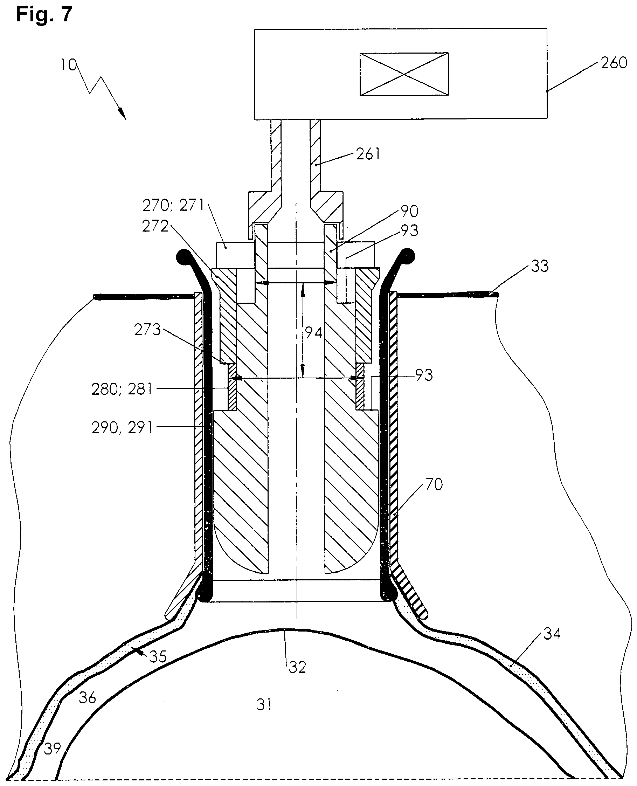

Figur 7 zeigt eine erfindungsgemäße Vorrichtung 10 in einer Gebrauchslage mit Dichtungen 280. Diese dichten geöffnetes Perikard 34 gegen die erfindungsgemäße Vorrichtung 10, so dass ein freier Fluidstrom, z.B. ein Gasstrom (nicht dargestellt) in das Perikard 34 oder aus diesem heraus verhindert wird.

Die vorgenannte Ausführungsform kann auch unabhängig von anderen Ausführungsformen verwendet werden.

Das Perikard 34 steht innerhalb seiner physiologischen Grenzen unter Druck (nicht dargestellt). Dieser, der Perikardbeutelinnendruck 36 ist gegenüber dem Umgebungsdruck (nicht dargestellt) im Intervall von ca. -15 mmHG bis 0 mmHG verringert. Innerhalb physiologischer Parameter ist der Perikardbeutelinnendruck 36 somit ein Unterdruck. Der Unterdruck füllt ein Innenvolumen 39 zwischen Herz 31 und Perikardinnenseite 35.

Problematisch ist, dass ein Öffnen des Perikards 34 in einem gravierenden Verlust von Pumpleistung des Herzens 31 enden kann.

Wird das Perikard 34 ungedichtet geöffnet, gleichen sich Umgebungsdruck und Perikardbeutelinnendruck 36 einander an, das Perikard 34 verliert seine innere Kompression, so dass das Herz 31 nicht mehr in einer funktionsunterstützenden Form gehalten wird. Die Ebene der Herzklappen (nicht dargestellt) und die Herzspitze sind durch Gewebe miteinander verbunden. Solange die Herzspitze 32 durch den negativen Perikardbeutelinnendruck 36, also einen Unterdruck in das Perikard 34 gesogen wird, hält dieser das Herz 31 in seiner typischen Kontur. Weil die Herzspitze 32 gehalten wird, bewegt sich die Ebene der Herzklappen in Relation zur Herzspitze. Ohne Unterdruck hingegen ist die Herzspitze 32 im Perikard 34 los gelagert, so dass sie sich in Relation zur Herzklappenebene bewegt. Dadurch wird die Funktionalität der Herzklappen (nicht dargestellt), u.a. ihr Hub verringert, in Folge dessen die Pumpleistung des Herzen 31 sinkt. Der Verlust an Kraftunterstützung entspricht dabei einem Verlust an Pumpleistung des Herzens 31 von über 20%.

Das Problem wird dadurch gelöst, dass der Perikardbeutelinnendruck 36 vor-, während und nach einer Behandlung unter Druck, also unterhalb des Umgebungsdrucks gehalten wird. Phänomenologisch ist die erfindungsgemäße Vorrichtung 10 daher gegen einen Anstieg des Perikardbeutelinnendruck 36 in Richtung Umgebungsdruck gedichtet. Er wird durch mindestens eine Vakuumpumpe 260 kontrolliert und gesteuert.

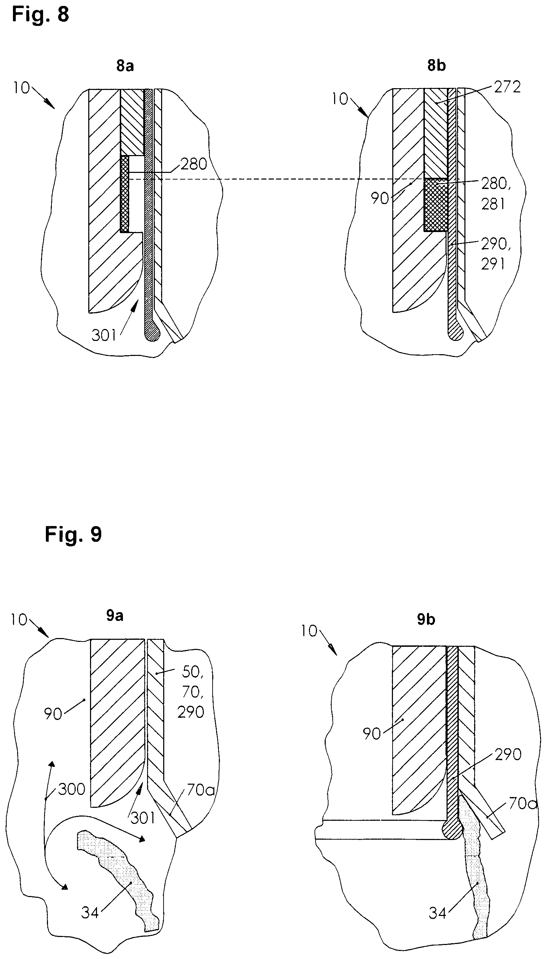

Die Ausführungsvariante in Figur 8 umfasst in der Gebrauchslage eine innere Röhre 70. Durch sie ist eine Stützstruktur 290 geführt.

In einer bevorzugten Ausführungsform ist die Stützstruktur 290 ein sogenannter Soft-Tissue Retraktor 291. Dieser wirkt vorteilhafterweise als Feuchtigkeitsgrenze und dichtet gegen Flüssigkeitsverlust.

Durch die Stützstruktur 290 ist ein Innenteil 90 beweglich geführt. Sein Freiheitsgrad kann in unterschiedlichen Ausführungsformen durch unterschiedliche Toleranzen vorbestimmt sein. Im Ausführungsfall normdichter Auslegung ist das Innenteil 90 gegen die Stützstruktur 290 auf Übergang gepasst, so dass beide Reibpartner auf Grund minimaler Reibung gegeneinander dichten. Als Einführungshilfe dient Gleitgel, welches auf das Innenteil 90 aufgetragen wird, bevor dieses durch den Soft-Tissue-Retraktor 291 geführt wird.

Das Innenteil 90 ist gestuft gefertigt, vorzugsweise sind dazu Innenteilbünde 93 aus dem vollen Material, z.B.: Titan, Aluminium oder Stahllegierungen ausgenommen, z.B. gefräst oder gedreht und durch die Haut 37 gesteckt. Die Innenteilbünde 93, (Stufen) variieren in ihrem Innenteildurchmesser 94, sie nehmen in der Figur 8 von oben nach unten zu, also in Richtung apikal zu. In einer bevorzugten Ausführungsform ist ein erster Innenteilbunddurchmesser 94 (Stufendurchmesser) kleiner als eine zweiter, apikal davon gedrehter Innenteilbunddurchmesser 94. Auf den zweiten Innenteilbunddurchmesser 94 ist ein elastischer Körper, eine Dichtung 280 gelagert. In vorteilhafter Ausgestaltung ist die Dichtung 280 eine Stopfbuchse 281. Sie ist in einer Ausführung z.B. aus einem Elastomer, wie Gummi oder Silikon gefertigt, mit Spiel auf den zweiten Innenteilbunddurchmesser 94 aufgeschoben. Über den zweiten Innenteilbund 93 ist zusätzlich ein Abstandhalter 272 geführt. Dieser ist axial entlang der Innenteillänge los gelagert, an seinem apikalen Ende, dem apikalen Abstandshalterende 273 berührt er in der gezeigten Gebrauchslage die Stopfbuchse 280. Weil der Abstandshalter 272 los gelagert ist, wird die Stellkraft eines mechanischen Mechanismus 270, in bevorzugter Ausführung die Anzugskraft einer Mutter 271 auf den Abstandhalter 272 geleitet, so dass die Stopfbuchse 281 komprimiert wird und sich gemäß ihrem elastischen Verhalten verformt. Ihre Ausdehnung wird sich axial verringern und radial zunehmen bis sie sich an die Innenseite des Soft-Tissue-Retraktor 291 erst anlegt und dann anpresst. Ein Einleiten einer Anzugskraft resultiert also in einem Dichten zwischen Innenteil 90 und Soft-Tissue-Retraktor 291 durch ein Einpressen eines Formkörpers.

Am ventralen Ende des Innenteil 90, d.h. in Figur 7 am oberen Bildende ist mittels eines Flunsches 261 eine Vakuumpumpe 260 angeschlagen. Diese hält in einer bevorzugten Ausführung vor, während und nach dem Einbringen der erfindungsgemäßen Vorrichtung 10 einen Druck unterhalb des Umgebungsdrucks auf Recht. Vorteilhafterweise kann eine Steuerung des Unterdrucks benutzt werden, um Herzkontraktion zu unterstützen. Diese Unterstützung kann in einer vorteilhaften Ausführung sowohl statische oder dynamische Druckänderungen oder beide vorgenannten Druckänderungen umfassen.

Die Aufgabe wird ebenfalls durch ein Bereitstellen einer Schleuse (nicht dargestellt) gelöst. Dazu kann eine Schleusenkammer (nicht dargestellt) auf das Innenteil 90 aufgesetzt oder angeflanschte werden, sobald Perikard 34 geöffnet ist.

Sobald das Innenteil 90 mit dem Perikard verhakt worden ist, kann eine Öffnungswerkzeug, z.B. ein Zackengreifer (nicht dargestellt) in das Innenteil 90 eingesetzt werden, sobald die alle Kammern (nicht dargestellt) der Schleuse auf den gleichen Unterdruck gebracht worden sind.

Nach Erreichen dieses Unterdruckes kann ein Schieber (nicht dargestellt) zwischen mindestens zwei Kammern (nicht dargestellt) geöffnet werden und ein benutztes Werkzeug aus der Kammer in die Schleusenkammer gezogen werden. Anschließend wird der Schieber zwischen beiden Kammern wieder in die Schließstellung gebracht. Danach lässt sich die Schleusenkammer öffnen und das benutzte Werkzeug gegen Anderes auswechseln. Nach Schließen der Schleusenkammer wird erneut der Unterdruck in der Schleusenkammer erzeugt, so dass der Schieber (nicht dargestellt) wieder geöffnet werden kann und das neue Werkzeug oder das Implantat (nicht dargestellt) durch die Konstruktion hindurch in Position gebracht werden kann. Vorteilhafterweise kann ein Werkzeugwechsel beliebig wiederholt werden.

Das Öffnungswerkzeug wird in einer bevorzugten Ausführungsform dann von außerhalb der Schleuse betätigt werden. Dazu stehen in der Technik kleine hydraulisch betätigte Hubzylinder und Zylinder für eine Drehbewegung zur Verfügung, die auch in den Rohren untergebracht werden können. Alternativ kann man diese hydraulischen Zylinder in die Rohrkonstruktion integrieren.

Im Nachgang einer Perikardöffnung 34a, z.B. im Hinblick auf eine Behandlung stellt sich das Problem, das Perikard 34 wieder zu verschließen. Diese Aufgabe wird durch die erfindungsgemäße Vorrichtung 10 dadurch gelöst, dass die Vakuumpumpe 260 ein Vakuum im Perikard 34 herstellt, dass geeignet ist dort einen bestimmten Unterdruck herzustellen, so dass das Herz 31 in eine physiologische Form gezogen wird. Durch das Ziehen des Unterdrucks löst die erfindungsgemäße Vorrichtung damit konsekutiv die Aufgabe eine Funktionalität der Herzklappenebene wiederherzustellen, bevor das Perikard 34 verschlossen wird. In einer bevorzugten Ausführungsform wird verschlossen, indem die Perikardöffnung 34a gelasert, vernäht, verklebt oder getackert wird.

Figur 8 zeigt eine Detailansicht einer erfindungsgemäßen Vorrichtung in zwei unterschiedlichen Zuständen: geschlossen und offen bzw. gedichtet und gasdurchlässig.

Figur 8a zeigt eine Detailansicht einer erfindungsgemäßen Vorrichtung 10 in einer gasdurchlässigen Gebrauchslage. Der Formkörper, die Stopfbuchse 280 ist frei von Kompressionskräften, so dass mindestens ein Spalt 301 resultiert.

Figur 8b zeigt eine Detailansicht einer erfindungsgemäßen Vorrichtung 10 in einer gasdichten Gebrauchslage. Die Dichtung 280, d.h. die Stopfbuchse 281 liegt am Soft-Tissue-Retraktor 291, einer Stützstruktur 290 an, so dass ein freier Gasstrom (nicht dargestellt) durch Spalte (vgl. Figur 8a) verhindert ist, das Innenteil 90 ist gegen den Soft-Tissue-Retraktor 291 gedichtet. Zur Dichtung drückt der Abstandshalter 272 die Stopfbuchse 281 zusammen.

Figur 9 zeigt eine Detailansicht einer erfindungsgemäßen Vorrichtung in zwei unterschiedlichen Zuständen: gasdurchlässig und gasdicht.

Figur 9a zeigt eine Detailansicht einer erfindungsgemäßen Vorrichtung 10 in einer gasdurchlässigen Gebrauchslage. Das Perikard 34 ist durchtrennt, so dass ein Gasstrom 300 in oder aus dem Perikard 34 heraus resultiert. Die Figur 9a zeigt drei Gasströme 300. Einer fließt auf einem ersten Weg durch mindestens eine erste Innenteilbohrung in das Perikard 34, auf einem zweiten Weg zwischen mindestens einer Außenseite des Innenteil 90 und einer Stützstruktur 290, einer Röhre 70 oder 50, auf einem dritten Weg zwischen Außenseite des Innenteils 90 und mindestens einem offenen Ende, d.h. einem Konus 70a.

Figur 9b zeigt eine Detailansicht einer erfindungsgemäßen Vorrichtung 10 in einer gasdichten Gebrauchslage. Das Perikard 34 ist zwischen der Stützstruktur 290, zwischen einem Soft-Tissue Retraktor und einem Konus 70a geklemmt und damit gedichtet.

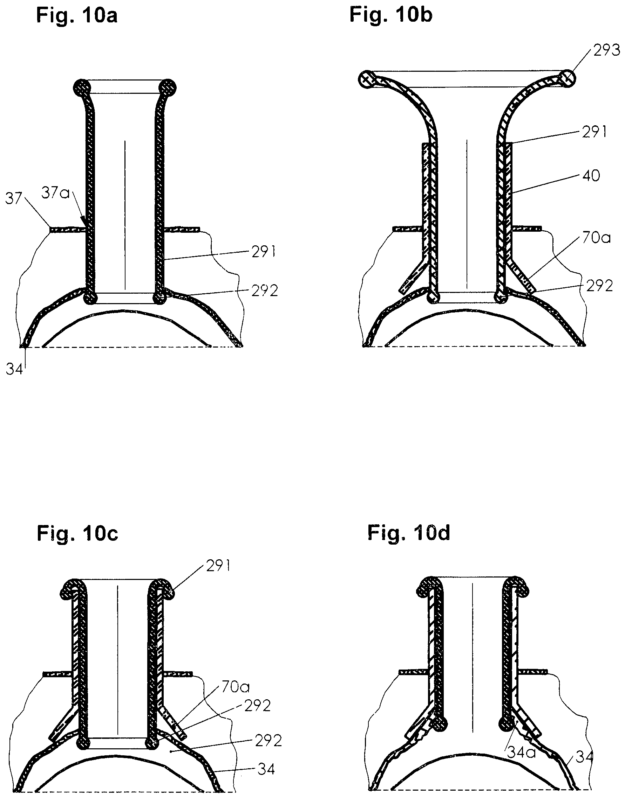

Figur 10 zeigt eine schematische Darstellung von Lagen einer erfindungsgemäßen Vorrichtung zum Einbringen eines Implantates (nicht dargestellt).

Die vorgenannte Ausführungsform kann auch unabhängig von anderen Ausführungsformen verwendet werden.

Figur 10a zeigt schematisch eine Öffnungslage an einer Behandlungsstelle. Ein Hautabschnitt 37 bzw. Haut 37 ist durchtrennt, so dass eine Einführungsöffnung 37a eröffnet ist. Sie wird z.B. durch einen Skalpellschnitt gesetzt. Durch die Einführungsöffnung 37a ist anschließend eine Soft-Tissue-Retraktor 291 vorschiebbar.

In einer bevorzugten Vorgehensweise endet dessen Vorschub, sobald er auf dem Perikard 34 aufliegt. Das Aufliegen kann manuell oder maschinell erfühlt, ertastet oder optisch geprüft werden. Wenn der Soft-Tissue-Retraktor 291 aufliegt, ist durch diesen ein Schneid-, oder Schubinstrument durch ihn führ- und vorschiebbar bis das Perikard 34 erreicht und durchtrennt ist. Alternativ kann das Perikard 34 mit mindestens einer Kanüle (nicht dargestellt), speziellem Werkzeug (nicht dargestellt), z.B. Ring-, Nut- oder Zackengreifer, einer Nadel (nicht dargestellt) oder einem Skalpell (nicht dargestellt) eröffnet werden. Durch das eröffnete Perikard 34 wird ein Ende des Soft-Tissue-Retraktor 291 durch ihn vorgeschoben bis dessen apikaler Spannring 292 hinter der Perikardöffnung klemmt.

Figur 10b zeigt schematisch eine geöffnete Behandlungsstelle mit einem eingeführten Soft-Tissue-Retraktor 291. Ein apikaler Spannring 292, ein ventraler Spannring 293 oder beiden des Soft-Tissue-Retraktor 291 werden manuell oder maschinell gefaltet, zusammengedrückt, geknautscht bis er oder sie durch mindestens ein Außenteil 40 oder eine innere-Röhre (nicht dargestellt) oder eine äußere-Röhre (nicht dargestellt) oder ein Steckverbund aus innerer und äußerer Röhre (vgl. Figur 6e) geführt werden können. In bevorzugter Ausführung ist das Außenteil ein sogenannter Hüllen-Spanntrichter. Ist der Soft-Tissue-Retraktor 291 durch einen Hüllen-Spanntrichter geführt, wird letzterer zum Perikard vorgeschoben bis dessen Trichtermund, ein Konus 70a berührt.

Figur 10c zeigt ein Spannen eines Soft-Tissue-Retraktor 291, um Perikard 34 zwischen einer Innenseite eines Konus 70a und einem apikalen Spannring 292 eines Soft-Tissue-Retraktor 291 zu klemmen. Dazu wird am ventralen Spannring 293, im Bild das obere Ende des Soft-Tissue Retraktor 291 gezogen. Mindestens Teile oder Abschnitte des apikalen Spannrings 292 nehmen Perikard 34 dabei mit bis dieses im Konus 70a klemmt.

Figur 10d zeigt eine gedichtete Perikardöffnung 34a. Zwischen einem Soft-Tissue-Retraktor 291 und einem Konus 70a klemmt Perikard 34, so dass ein Fließen von Fluidströmen, insbesondere Gasströmen (nicht dargestellt) verhindert ist, dass Spalte (nicht dargestellt) geschlossen sind.

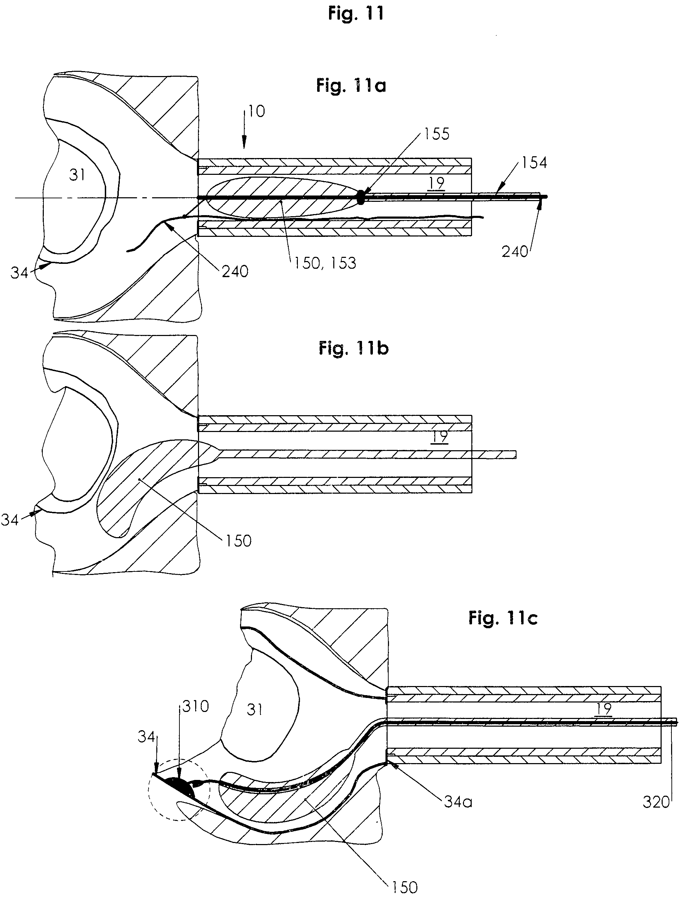

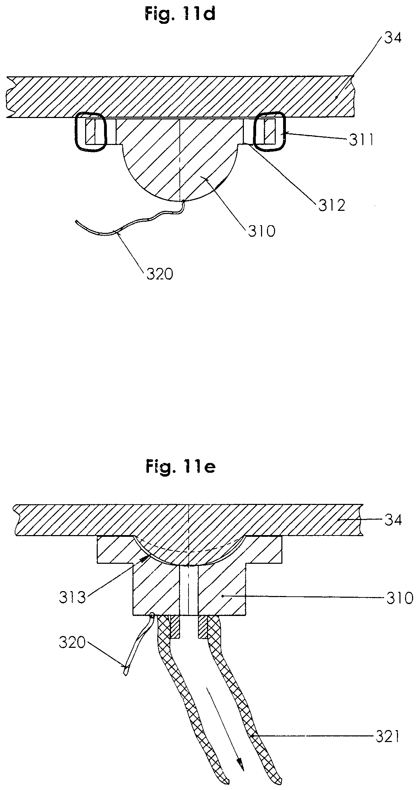

Figur 11 zeigt eine schematische Darstellung von Lagen eines Implantats.

Figur 11a zeigt eine Lage eines Implantats 150 innerhalb der erfindungsgemäßen Vorrichtung 10. Das Implantat 150 ist mit einer Implantatführung 154 verbunden. In bevorzugte Ausführungsform ist zwischen dem Implantat 150 und der Implantatführung 154 eine Implantatführung als Bruchstelle 155 vorgesehen. Alternativ kann diese durch stärkeres Wackeln oder Drehen zerbrochen werden, so dass das Implantat 150 von der Implantatführung 154 getrennt wird.

In einer bevorzugten Ausführungsform ist durch das Implantat 150, die Bruchstelle 155 und die Implantatführung 154 ein durch alle drei vorgenannten Bauteile durchgehender Durchlass ausgearbeitet. Durch diesen kann Druckluft oder Fluid in das Implantat 150 ein-, oder herausgebracht werden.

In einer bevorzugten Ausführungsform ist ein Führungsdraht 240 durch den durchgehenden Durchlass geführt, der an mindestens einer Stelle mit dem Implantat 150 verbunden ist. Die Verbundstelle kann ein Knoten, ein Schweiß-, oder ein Klebpunkt sein. Weil der Führungsdraht 240 an das Implantat 150 damit angeschlagen ist, kann es durch den Führungsdraht 240 geführt werden.

In einer bevorzugten Ausführungsform ist das Implantat 150 ein Kissen 153, dessen Volumen durch ein Zu-, und Abführen von Druckluft über die Zeit veränderbar ist. Alternativ kann ein Gas verwendet werden.

Alternativ kann durch den Vorrichtungstunnel 19 ein Führungsdraht 240 eingebracht werden, welcher von außen an das Implantat 150 angeschlagen ist.

Mittels des Führungsdrahts 240 kann das Implantat 150 in Richtung Herz 31 bis zum Perikard 34 vorgeschoben werden

Figur 11b zeigt eine Lage eines Implantats 150 innerhalb der erfindungsgemäßen Vorrichtung oder einer anderen beliebigen rohrartigen Führungskonstruktion. Das Implantat 150 wird alternativ durch einen Führungsdraht (vgl. Fig.11a) oder durch eine Implantatführung (nicht dargestellt) in Richtung des Perikards 34 durch den Vorrichtungstunnel 19 vorgeschoben.

Figur 11c zeigt eine alternative Lage eines Implantats 150 im Vergleich zu Fig. 11b zu dessen temporärer Unterstützung. Dazu ist das Perikard 34 eröffnet, so dass das Implantat 150 durch eine Perikardöffnung hindurchgeschoben werden kann.

Dabei besteht die Aufgabe, dass das Implantat 150 möglichst tief in das Perikard 34 hineingebracht werden soll. Diese Aufgabe wird durch ein Verfahren dadurch gelöst, dass ein Implantatanker 310 in das Perikard 34 eingebracht und fixiert wird.

Das Verfahren umfasst dazu die Schritte Haut (nicht dargestellt) zu eröffnen, eine erfinderische Vorrichtung durch die Öffnung bis zum Perikard 34 vorzuschieben, das Perikard 34 zu greifen und zu öffnen, einen Implantatanker 310 durch die erfinderische Vorrichtung und durch die Perikardöffnung 34a vorzuschieben und zwischen an einer Innenseite des Perikard 34, zwischen Perikard 34 und Herz 31 anzuordnen, mindestens einen Führungsdraht 320 zurückzuführen und durch einen Durchlass eines Implantat 150 durchzuführen und das Implantat 150 entlang des Führungsdrahts 320 durch einen Vorrichtungstunnel 19 des Führungsdrahts 320 bis zum Implantatanker 310 vorzuführen.

Vorteilhafterweise kann mindestens ein Implantat 150 sicher zwischen Herz 31 und Herzbeutel 34 positioniert werden, so dass ein Implantat 150 an eine vordefinierte Stelle gebracht werden kann.

In einer bevorzugten Ausführungsform wird als Druckmittler des Implantats 150 eine Flüssigkeitssäule verwendet. Diese löst die Aufgabe, Druckänderungen fast unmittelbar zu übertragen. Wenn im Implantat zugleich der Herzschlag gemessen wird, kann über eine rechnergestützte Steuerung die Unterstützung des Herzens optimal gesteuert werden.

Die in Figur 11c genannte Ausführungsform kann auch unabhängig von anderen Ausführungsformen verwendet werden.

Figur 11d zeigt eine Detailansicht mit einem Implantatanker 310. Der Implantatanker 310 ist durch mindestens eine Schnur 311 mittels mindestens einer Öse 312 mit dem Perikard 34 vernäht. In einer besonders vorteilhaften Ausführungsform ist der Implantatanker 310 auf seinem Umfang mittels einer Mehrzahl an Ösen 312 mit dem Perikard 34 vernäht. Ein Führungsdraht 320 ist an ihn angeschlagen.

Figur 11e zeigt ein Detail mit einem Implantatanker 310. Dieser ist mittels eines Unterdrucks an das Perikard 34 herangesaugt. Dazu ist ein Führungssaugschlauch 321 an den Implantatanker 310 angeflanscht, so dass durch diesen, Luft bzw. Fluid aus mindestens einem Implantatdom 313 im Implantatanker 310 gefördert, z.B. gepumpt werden kann. Dieses hat den Vorteil, dass der Implantatanker 310 an das Perikard 34 herangesaugt wird. Ein Führungsdraht 320 bzw. eine Führungsseil (nicht dargestellt) oder eine Führungsschnur (nicht dargestellt) kann zusätzlich am Implantatanker 310 angeschlagen sein.

### Bezugsziffernliste

- 10: Vorrichtung
- 12: Handseitiges Vorrichtungsende
- 13: Vorrichtungsspitze
- 15: Axiale Richtung
- 16: Volumen
- 19: Vorrichtungstunnel

- 20: Ringgreifer

- 31: Herz
- 32: Herzspitze
- 33: Herz

- 34: Perikard
- 34a: Perikardöffnung
- 35: Perikardinnenseite
- 36: Perikardbeutelinnendruck

- 37: Haut
- 37a: Einführungsöffnung
- 39: Innenvolumen

- 40: Außenteil
- 41: Außenteilende
- 42: Außenteilendlänge
- 43: Außenteilendabsatz
- 44: Außenteilendfläche

- 50: Äußere Röhre
- 51: Äußerer Außenröhrendurchmesser
- 52: Innerer Außenröhrendurchmesser
- 53: Außenröhrenende
- 54: Außenröhrenendfläche
- 56: Außenröhrenbund
- 59: Außenröhrenendflächenprofil
- 59a: Außenröhrenendflächenkerben

- 60: Äußeres Rändelrad
- 61: Rotationsrichtung äußeres Rändelrad

- 70: Innere Röhre
- 70a: Konus
- 71: Äußerer Innenröhrendurchmesser
- 72: Innerer Innenröhrendurchmesser
- 74: Innenröhrenendfläche
- 76: Innenröhrenbund
- 78: Innenröhrenendflächenprofil

- 80: Inneres Rändelrad
- 81: Rotationsrichtung inneres Rändelrad

- 90: Innenteil
- 93: Innenteilbund
- 94: Innenteilbunddurchmesser

- 100: Stift
- 101: Äußere Stiftdurchmesser
- 102: Durchgangsbohrung
- 103: Durchgangsbohrungsdurchmesser
- 104: Stiftbund
- 105: Stiftspitze
- 106: Stiftloch
- 107: Stiftlochdurchmesser
- 108: Stiftknauf
- 109: Stiftlänge

- 110: Fühler
- 111: Fühlerschaft
- 112: Fühlerkopf

- 120: Federelement

- 130: Sicherung
- 131: Sicherungsnut
- 132: Sicherungsring

- 150: Implantat
- 153: Kissen
- 154: Implantatführung
- 155: Implantatführungsbruchstelle

- 200: Menschlicher Körper
- 201: Körperzugangsöffnung
- 202: Eindringtiefe
- 203: Arbeitstiefe

- 220: Zacke
- 220a: Zackengreifer
- 220b: Zackengreiferschaft
- 221: Zackenmittellinie
- 222: Zackentasche
- 223: Anstellwinkel
- 224: Freilauf
- 225: Greifrichtung

- 230: Nutgreifer
- 231.1: Außenröhrennut
- 231.2: Innenröhrennut
- 233.3: Innenteilnutgreifer
- 240: Führungsdraht

- 250: Punktionsnadel

- 260: Vakuumpumpe
- 261: Flunsch

- 270: Mechanischer Mechanismus
- 271: Mutter
- 272: Abstandshalter
- 273: Apikales Abstandhalterende

- 280: Dichtung
- 281: Stopfbuchse

- 290: Stützstruktur
- 291: Soft-Tissue Retractor
- 292: Apikaler Spannring
- 293: Ventraler Spannring
- 294: Feststellabsatz

- 300: Gasstrom
- 301: Spalt

- 310: Implantatanker
- 311: Schnur
- 312: Öse
- 313: Implantatdom

- 320: Führungsdraht
- 321: Führungssaugschlauch

## Patentansprüche

1. Vorrichtung zum Greifen von Perikard umfassend
- mindestens eine innere Röhre (70) und mindestens eine äußere Röhre (50),
- wobei die äußere Röhre mindestens einen äußeren Durchmesser (51) und einen inneren Außenröhrendurchmesser (52) aufweist und
- wobei die innere Röhre mindestens einen inneren Durchmesser (72) und einen äußeren Innenröhrendurchmesser (71) aufweist,
- wobei der äußere Durchmesser größer ausgebildet ist als der innere Durchmesser und der innere Außenröhrendurchmesser größer als der äußere Innenröhrendurchmesser ausgebildet ist, und
- die innere Röhre an mindestens einem Innenröhrenende mindestens eine Innenröhrenendfläche (74) aufweist,
- und wobei die äußere Röhre an mindestens einem Außenröhrende (53) mindestens eine Außenröhrenendfläche (54) aufweist,
und wobei die Außenröhrenendfläche mindestens eine zweite Oberflächenstruktur (59) aufweist,
**dadurch gekennzeichnet, dass**
die innere Röhre beweglich in der äußeren Röhre angeordnet ist und wobei die Innenröhrenendfläche mindestens eine erste Oberflächenstruktur (78) aufweist und die erste Oberflächenstruktur und die zweite Oberflächenstruktur unterschiedlich ausgebildet sind, wobei die innere Röhre drehbar in der äußeren Röhre angeordnet ist.

2. Vorrichtung nach Anspruch 1, **dadurch gekennzeichnet, dass** mindestens ein Innenteil (40) vorhanden ist, wobei das Innenteil (40) in der inneren Röhre beweglich angeordnet ist.

3. Vorrichtung nach mindestens einem der vorhergehenden Ansprüche, **dadurch gekennzeichnet, dass** in mindestens einer Durchgangsbohrung (401) des Innenteils (40) mindestens eine Fühlersonde (403b) vorhanden ist.

4. Vorrichtung nach mindestens einem der vorhergehenden Ansprüche, **dadurch gekennzeichnet, dass** zwischen einer Fläche des Innenteils (402) und einer Fläche des Fühlersondenkopfs (403c) mindestens ein Federelement (50) angeordnet ist.

5. Vorrichtung nach mindestens einem der vorhergehenden Ansprüche, wonach mindestens eine Röhre flexibel, insbesondere ein Schlauch ist.

## Claims

1. Device for gripping pericardium comprising
- at least one inner tube (70) and at least one outer tube (50),
- wherein the outer tube has at least one outer diameter (51) and an inner outer tube diameter (52), and
- said inner tube having at least one inner diameter (72) and an outer inner tube diameter (71), wherein
- the outer diameter is larger than the inner diameter and the inner outer tube diameter being larger than the outer inner tube diameter, and
- the inner tube has at least one inner tube end surface (74) on at least one inner tube end,
- and wherein the outer tube has at least one outer tube end surface (54) on at least one outer tube end (53),
and wherein the outer tube end surface has at least a second surface structure (59),
**characterized in that**
the inner tube is movably arranged in the outer tube and wherein the inner tube end surface has at least a first surface structure (78) and the first surface structure and the second surface structure are formed differently, wherein the inner tube is rotatably arranged in the outer tube.

2. Device according to claim 1, **characterized in that** at least one inner part (40) is provided, said inner part (40) being movably arranged in said inner tube.

3. Device according to at least one of the preceding claims, **characterized in that** at least one sensor probe (403b) is present in at least one through bore (401) of the inner part (40).

4. Device according to at least one of the preceding claims, **characterized in that** at least one spring element (50) is arranged between a surface of the inner part (402) and a surface of the sensor probe head (403c).

5. Device according to at least one of the preceding claims, wherein at least one tube is flexible, in particular a hose.

## Revendications

1. Dispositif de préhension du péricarde comprenant
- au moins un tube interne (70) et au moins un tube externe (50), dans lequel
- le tube externe ayant au moins un diamètre externe (51) et un diamètre intérieur de tube externe (52), et
- le tube interne ayant au moins un diamètre interne (72) et un diamètre extérieur de tube interne (71),
- le diamètre externe est plus grand que le diamètre interne et le diamètre extérieur du tube interne est plus grand que le diamètre intérieur du tube externe, et
- le tube interne présente au moins une surface d'extrémité de tube interne (74) à au moins une extrémité de tube interne,
- et dans lequel le tube externe présente au moins une surface d'extrémité de tube externe (54) à au moins une extrémité de tube externe (53),
et dans lequel la surface d'extrémité du tube externe comprend au moins une deuxième structure de surface (59),
**caractérisé en ce que**
le tube interne est disposé de manière mobile dans le tube externe et dans lequel la surface d'extrémité du tube interne comprend au moins une première structure de surface (78) et la première structure de surface et la deuxième structure de surface sont formées différemment, le tube interne étant disposé de manière rotative dans le tube externe.

2. Dispositif selon la revendication 1, **caractérisé en ce qu'**il y a au moins une partie intérieure (40), la partie intérieure (40) étant disposé de manière mobile dans le tube interne.

3. Dispositif selon au moins l'une des revendications précédentes, **caractérisé en ce qu'**au moins une sonde de détection (403b) est présente dans au moins un trou de passage (401) de la partie intérieure (40).

4. Dispositif selon au moins l'une des revendications précédentes, **caractérisé en ce qu'**au moins un élément élastique (50) est disposé entre une surface de la partie intérieure (402) et une surface de la tête de la sonde de détection (403c).

5. Dispositif selon au moins une des revendications précédentes, selon lequel au moins un tube est flexible, en particulier est un tuyau.
